# EUROPEAN PATENT APPLICATION

(11) **EP 4 628 892 A1**
(43) Date of publication of application: **08.10.2025**
(21) Application number: 23898303.5
(22) Date of filing: 29.11.2023
(51) Int. Cl.: G01N 33/50

(54) **INHIBITOR OF ACTIVITY OR EXPRESSION OF NGR1 PROTEIN FOR IMPROVING CYTOLYTIC ACTIVITY BY PROMOTING FORMATION OF IMMUNOLOGICAL SYNAPSES OF IMMUNE CELLS**

(30) Priority: 29.11.2022 KR 20220162743
(71) Applicant: Korea Research Institute of Bioscience and Biotechnology, Daejeon 34141 (KR)
(72) Inventor: KIM, Tae-Don, Daejeon 34141 (KR); OH, Se-Chan, Daejeon 34141 (KR)
(74) Representative: Ipsilon
(86) International application number: PCT/KR2023/019461
(87) International publication number: WO 2024/117777

(57) **Abstract**

The present invention relates to a target for stably forming immunological synapses and improving cytolytic activity in immune cells, and use thereof, and uses an inhibitor of NgR1 protein activity or expression, and thus can improve the stability of synapse formation and can improve cytolytic activity or cytotoxicity, and uses the NgR1 protein as a target so as to measure activity or expression level, and thus can screen for novel anticancer agents. In addition, the present invention can promote the formation of immunological synapses, which are points of contact between immune cells and cancer cells, and can also regulate, in organs and tissue in the body, cell-to-cell contact between various cells that are the same or different from each other, including immune cells, cancer cells, nerve cells and the like, and thus can regulate cell functions.

## Description

### TECHNICAL FIELD

### [CROSS-REFERENCE TO RELATED APPLICATIONS]

This application claims priority from Korean Patent Application No. 10-2022-0162743, filed on November 29, 2022, the disclosure of which is incorporated herein by reference in its entirety.

### [TECHNICAL FIELD]

The present invention relates to an inhibitor of activity or expression of NgR1 protein for enhancing cytolytic activity by promoting formation of an immunological synapse of immune cells.

### BACKGROUND ART

Cell-to-cell contact, where cells physically come into contact with each other, is essential for transmitting strong and fast signals between cells identical to or different from each other. Cell-to-cell contact, which is one of the types of communication between cells, regulates the function of the cells through binding of molecules expressed on a cell surface and regulation of activity of continuous intracellular signaling molecules. Cell-to-cell contact is a priority for cell signaling using cell surface molecules. Errors in the formation and process of the cell-to-cell contact may lead to various diseases including cancer, autoimmune diseases, and metabolic diseases. Therefore, a deeper understanding of the function of the cells through cell-to-cell contact may help treat diseases more effectively.

Meanwhile, cell-to-cell contact may occur between various cells, and may occur between the same or different types of cells that make up organs and tissue in the body, including between neurons, between cancer cells, between immune cells, and between cancer cells and immune cells. Cell-to-cell contact may be described as a synapse formed by neurons of the nervous system, and the synapse of the neurons has a characteristic of regulating the function of neurons by regulating activity of a nerve signal by transmitting a neurotransmitter based on the cell-to-cell contact. Abnormal synapse formation may cause dysfunction of normal neurons due to dysregulation of the activity of neuron signal, which may induce various neurophysiological and pathological diseases. In the nervous system, guidance cues, including attractive and repulsive cues, are known to regulate axon growth or synapse formation in neurons. Attractive cues promote the axon growth and synapse formation in neurons, whereas repulsive cues hinder this. When neurons are damaged, nerve regeneration and synapse formation for normal neuronal function are inhibited by the repulsive cues. Nogo receptor 1 (NgR1) is a representative factor that recognizes NogoA, which is a ligand, and causes cytoskeleton shrinkage of neurons, thereby inhibiting nerve regeneration and synapse formation, NgR1 is limited to the cell-to-cell contact between neurons and the function thereof has been well studied, but the function and biological significance of the cell-to-cell contact between immune cells, cancer cells, and other diverse cells require deep studies.

Another representative example of cell-to-cell contact is an immunological synapse, which originates from a synapse of the nervous system that shares similar characteristics of cell-to-cell contact and signaling. The immunological synapse is also involved in regulating various functions of immune cells through intracellular signaling based on cell-to-cell contact. In particular, it is essential for a process by which immune cells recognize and eliminate target cells such as cancer cells or virus-infected cells. Cell surface molecules of immune cells recognize cell surface molecules of target cells and activate or inhibit signals transmitted into the immune cells, thereby regulating cytolytic activity to eliminate the target cells. Prior to the regulation of intracellular signaling activity of immune cells that recognize target cells, cell-to-cell contact between immune cells and cancer cells and formation of an immunological synapse through this is a priority process.

Among the cells that make up the immune system, in particular, natural killer cells (NK cells) are effector cells that act at the front of the in vivo immune system defense mechanism, such as a function of eliminating tumor cells, bacteria, intracellular parasites, or virus-infected host cells without pre-sensitization by an antigen, rejecting inadequate bone marrow transplantation, and regulating the immune response of T cells.

Among the various functions of these natural killer cells, in particular, the ability to selectively kill cancer cells has been discovered, and accordingly, many related studies have been conducted. In the case of cancer patients, the number or activity of natural killer cells in the peripheral blood was reduced compared to normal people, and in animal experiments, it was found that a risk of cancer occurrence and cancer metastasis increases when the activity of natural killer cells is reduced.

In addition, natural killer cells play an important role in the innate immune response against host-infected pathogens or cancer cells, as well as in the acquired immune response through cytokine secretion. A primary mechanism used for cytolysis, in which natural killer cells kill target cells, is to secrete cytolytic granules such as perforin and granzyme B into the target cells through an immunological synapse, the secreted perforin makes a hole in a wall of the target cell, and granzyme entered the target cell through the hole induces apoptosis of the target cell. Normal immunological synapse formation provides the basis for effective killing of target cells, but abnormal immunological synapse formation prevents successful target cell lysis by immune cells. As such, as natural killer cells have been confirmed to play a crucial role in a carcinogenic process in which cells become malignant or in the early stage of defense mechanisms in viral infections, and based on these studies, immune cell therapy using immune cells such as natural killer cells, T cells, and natural killer T cells has emerged in a cancer treatment and an immune disease treatment, there is a need to discover a novel substance that enhances cytolytic activity or cytotoxicity by promoting cell-to-cell contact and formation of an immunological synapse of immune cells.

### DISCLOSURE OF THE INVENTION

### TECHNICAL PROBLEM

An object of the present invention is to provide a novel antibody or an antigen-binding fragment thereof that specifically binds to NgR1 protein.

Another object of the present invention is to provide a gene encoding the antibody or the antigen-binding fragment thereof.

Still another object of the present invention is to provide a recombinant expression vector containing the gene.

Still another object of the present invention is to provide a transformant in which the recombinant expression vector is introduced into a host cell.

Still another object of the present invention is to provide a method for producing the antibody or the antigen-binding fragment thereof, the method including culturing the transformant.

Still another object of the present invention is to provide a novel small interfering RNA that specifically binds to NgR1 mRNA.

Still another object of the present invention is to provide a novel antisense oligonucleotide that specifically binds to NgR1 mRNA.

Still another object of the present invention is to provide a composition that enhances cytolytic activity of immune cells by promoting formation of an immunological synapse of the immune cells.

Still another object of the present invention is to provide a chimeric antigen receptor containing the antibody or antigen-binding fragment thereof.

Still another object of the present invention is to provide an immune cell expressing the chimeric antigen receptor.

Still another object of the present invention is to provide a method for diagnosing prognosis of a cancer patient or providing information for determining a treatment strategy.

Still another object of the present invention is to provide a method for screening an anticancer agent.

### TECHNICAL SOLUTION

An aspect of the present invention provides an antibody or an antigen-binding fragment thereof that specifically binds to NgR1 protein, the antibody or antigen-binding fragment thereof including: a heavy chain variable region containing a CDR1-H having an amino acid sequence of SEQ ID NO: 1, SEQ ID NO: 41, SEQ ID NO: 43, or SEQ ID NO: 45, a CDR2-H having an amino acid sequence of SEQ ID NO: 3, SEQ ID NO: 47, or SEQ ID NO: 49, and a CDR3-H having an amino acid sequence of SEQ ID NO: 5, SEQ ID NO: 7, SEQ ID NO: 9, SEQ ID NO: 51, SEQ ID NO: 53, SEQ ID NO: 55, or SEQ ID NO: 57; and a light chain variable region containing a CDR1-L having an amino acid sequence of SEQ ID NO: 23, SEQ ID NO: 79, or SEQ ID NO: 81, a CDR2-L having an amino acid sequence of SEQ ID NO: 25, SEQ ID NO: 83, or SEQ ID NO: 85, and a CDR3-L having an amino acid sequence of SEQ ID NO: 27, SEQ ID NO: 87, or SEQ ID NO: 89.

Another aspect of the present invention provides an antibody or an antigen-binding fragment thereof that specifically binds to NgR1 protein, the antibody or the antigen-binding fragment thereof including: a heavy chain variable region having an amino acid sequence of SEQ ID NO: 11, SEQ ID NO: 13, SEQ ID NO: 15, SEQ ID NO: 59, SEQ ID NO: 61, SEQ ID NO: 63, SEQ ID NO: 65, or SEQ ID NO: 67; and a light chain variable region having an amino acid sequence of SEQ ID NO: 29, SEQ ID NO: 31, SEQ ID NO: 33, or SEQ ID NO: 91.

Still another aspect of the present invention provides an antibody or an antigen-binding fragment thereof that specifically binds to NgR1 protein, the antibody or the antigen-binding fragment thereof including: a heavy chain having an amino acid sequence of SEQ ID NO: 17, SEQ ID NO: 19, SEQ ID NO: 21, SEQ ID NO: 69, SEQ ID NO: 71, SEQ ID NO: 73, SEQ ID NO: 75, or SEQ ID NO: 77; and a light chain having an amino acid sequence of SEQ ID NO: 35, SEQ ID NO: 37, SEQ ID NO: 39, or SEQ ID NO: 94.

The antigen-binding fragment of the antibody may be a single chain variable fragment (scFv).

Still another aspect of the present invention provides a gene encoding the antibody or the antigen-binding fragment thereof.

Still another aspect of the present invention provides a recombinant expression vector containing the gene.

Still another object of the present invention provides a transformant in which the recombinant expression vector is introduced into a host cell.

Still another object of the present invention provides a method for producing an antibody or an antigen-binding fragment thereof that specifically binds to NgR1 protein, the method including culturing the transformant.

Still another object of the present invention provides a small interfering RNA having a polynucleotide sequence of any one of SEQ ID NO: 99 to SEQ ID NO: 106 and specifically binding to NgR1 mRNA.

Still another object of the present invention provides an antisense oligonucleotide having a polynucleotide sequence of any one of SEQ ID NO: 107 to SEQ ID NO: 110 and specifically binding to NgR1 mRNA.

Still another object of the present invention provides a composition for enhancing cytolytic activity of immune cells, the composition containing, as an active ingredient, one or more selected from the group consisting of the antibody or the antigen-binding fragment thereof, the small interfering RNA, and the antisense oligonucleotide.

The composition may enhance the cytolytic activity of the immune cells by enhancing cytolytic activity of target cells (for example, cancer cells and the like) of immune cells through promotion of formation of an immunological synapse and/or regulation of the immune cells, thereby inhibiting growth of the target cells and/or inducing apoptosis of the target cells.

The composition may be a pharmaceutical composition for preventing or treating an NgR1-associated disease.

Specifically, the NgR1-associated disease may be one or more selected from the group consisting of cancer, multiple sclerosis, ALS, Huntington's disease, Alzheimer's disease, Parkinson's disease, diabetic neuropathy, stroke, traumatic brain injury, and spinal cord injury, but is not limited thereto, and may include all NgR1-associated diseases.

The cancer may be one or more selected from the group consisting of skin cancer, breast cancer, uterine cancer, esophageal cancer, stomach cancer, brain tumor, glioblastoma, colon cancer, rectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, blood cancer, thymus cancer, urethral cancer, and bronchial cancer, but is not limited thereto, and all diseases belonging to carcinoma may be included.

The composition may be an adjuvant for anticancer therapy.

The adjuvant may enhance an anticancer treatment effect by activating an immune function of immune cells by enhancing the activity of the immune cells through promotion of formation of an immunological synapse of the immune cells and/or regulation of the immune cells.

The composition may further contain an immuno-oncology agent.

The immune cells may be natural killer cells, T cells, or natural killer T cells.

The immuno-oncology agent may be one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-CD73, anti-ICOS, anti-CD40L, anti-BTLA, anti-TCR, anti-TIGIT, anti-VISTA, anti-TIGIT, and anti-NKG2A, but is not limited thereto, and any known immuno-oncology agents may be included.

The composition may further contain NEP1-40.

The NEP1-40 may have an amino acid sequence of SEQ ID NO: 111.

Still another aspect of the present invention provides a chimeric antigen receptor (CAR) containing the antibody or antigen-binding fragment thereof.

Still another aspect of the present invention provides a method for diagnosing prognosis of a cancer patient or providing information for determining a treatment strategy, the method including: measuring an expression level of Nogo protein in cancer cells isolated from the cancer patient; and comparing the expression level with an expression level of Nogo protein in normal cells.

Still another aspect of the present invention provides a method for screening an anticancer agent, the method including: treating a candidate substance to immune cells; measuring an expression level or activity level of NgR1 protein in the immune cells treated with the candidate substance; and comparing the measured expression level or activity level of the NgR1 protein with an expression level or activity level of NgR1 protein in immune cells not treated with the candidate substance.

The method for screening an anticancer agent may further include determining the candidate substance as an anticancer agent when the expression level or activity level of the NgR1 protein measured in the immune cells treated with the candidate substance is lower than the expression level or activity level of the NgR1 protein in the immune cells not treated with the candidate substance.

### ADVANTAGEOUS EFFECTS

In the present invention, NgR1 is identified as a factor affecting formation of an immunological synapse and cytolytic activity of immune cells, and it is confirmed that, when NgR1 protein is overexpressed or overactivated, cell-to-cell contact, formation of an immunological synapse, and cytolytic activity (cytotoxicity) of immune cells are reduced, while the cell-to-cell contact, the formation of an immunological synapse, and the cytolytic activity (cytotoxicity) of the immune cells against cancer cells may be enhanced by inhibiting the expression or activity of the NgR1 protein. Accordingly, there is an advantage in that it is possible to determine a treatment strategy to further improve a cancer treatment effect by inhibiting the expression or activity of NgR1 protein in immune cells, or to screen an inhibitor that may inhibit the expression or activity of NgR1 protein.

Furthermore, in the present invention, specific substances that may effectively inhibit the activity or expression of NgR1 protein, and may thus significantly enhance cell-to-cell contact, formation of an immunological synapse, and cytolytic activity of immune cells against cancer cells, are screened and optimized; thus, it is expected that these inhibitors may be usefully utilized for enhancing the cytolytic activity of the immune cells and for anticancer applications.

However, the effects of the present invention are not limited to the effects mentioned above, and other effects that are not mentioned may be obviously understood by those skilled in the art from the following description.

### BRIEF DESCRIPTION OF THE DRAWINGS

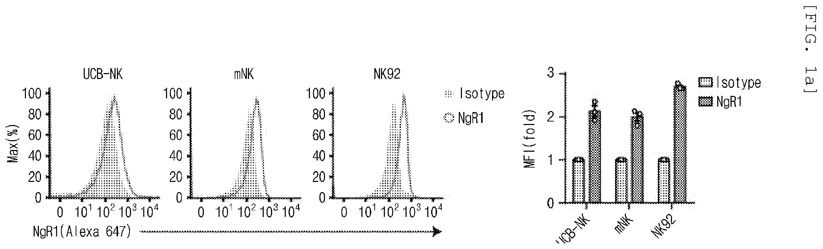

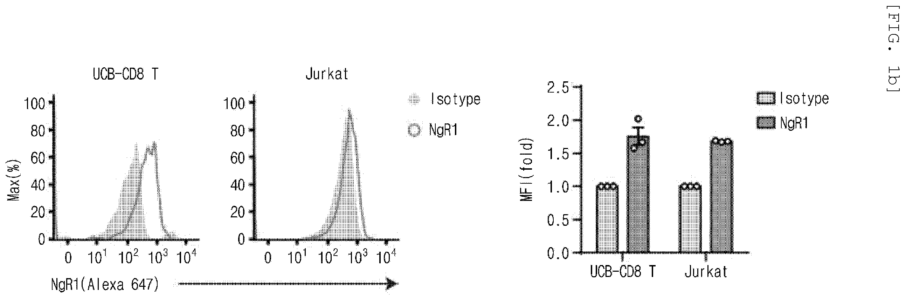

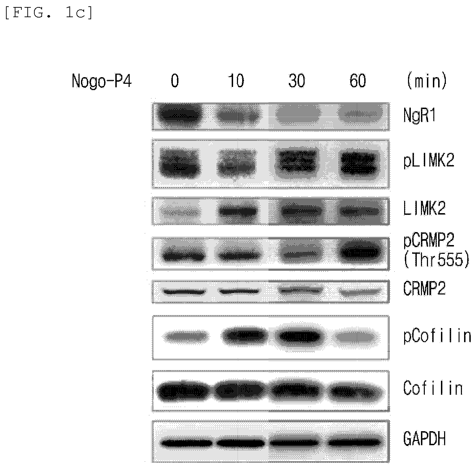

FIG. 1A illustrates the results of confirming whether NgR1 protein is expressed also in natural killer cells, FIG. 1B illustrates the results of confirming whether NgR1 protein is also expressed in T cells, and FIG. 1C illustrates the results confirming that the NgR1 protein expressed in natural killer cells also activates intracellular downstream signaling by a ligand thereof (Nogo protein).

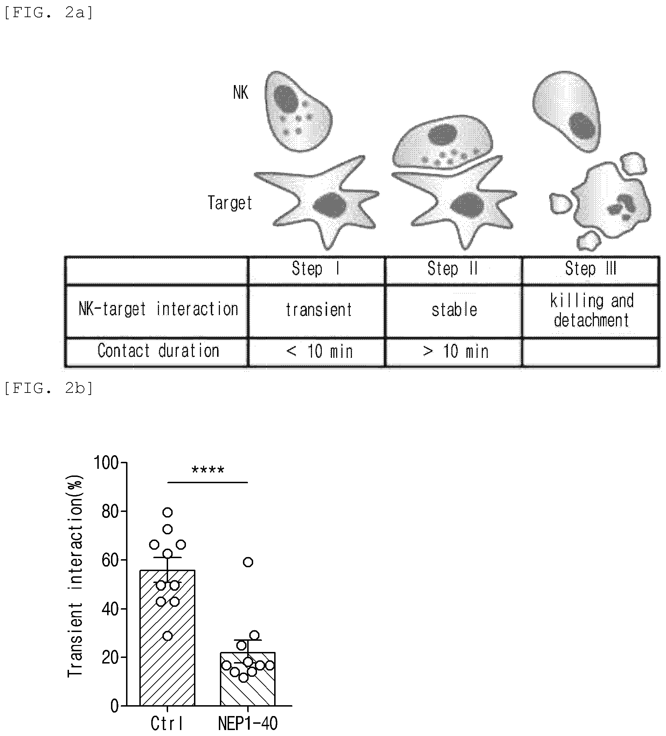

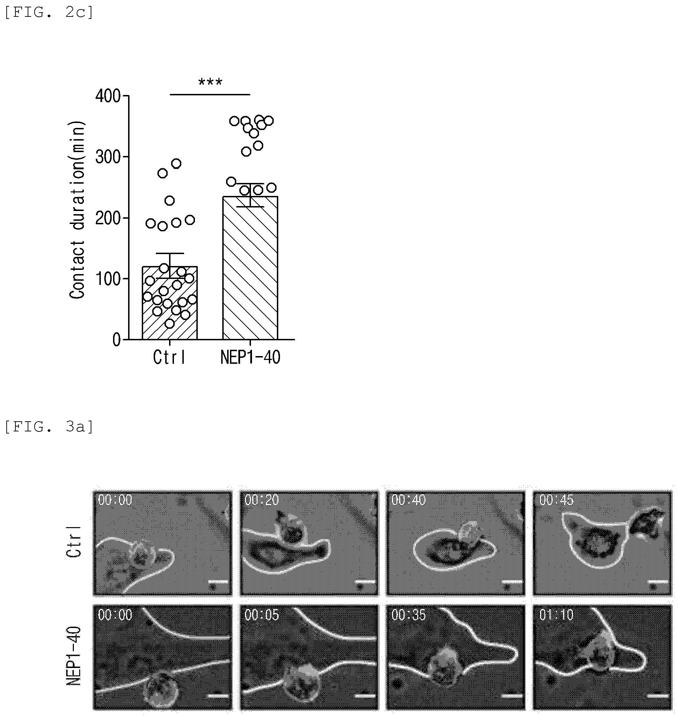

FIG. 2A illustrates each step of a process of contact between natural killer cells and target cells, and FIGS. 2B and 2C illustrate the results of confirming changes in ratio and contact duration in a transient contact step of the natural killer cells and the target cells according to inhibition of the activity of NgR1.

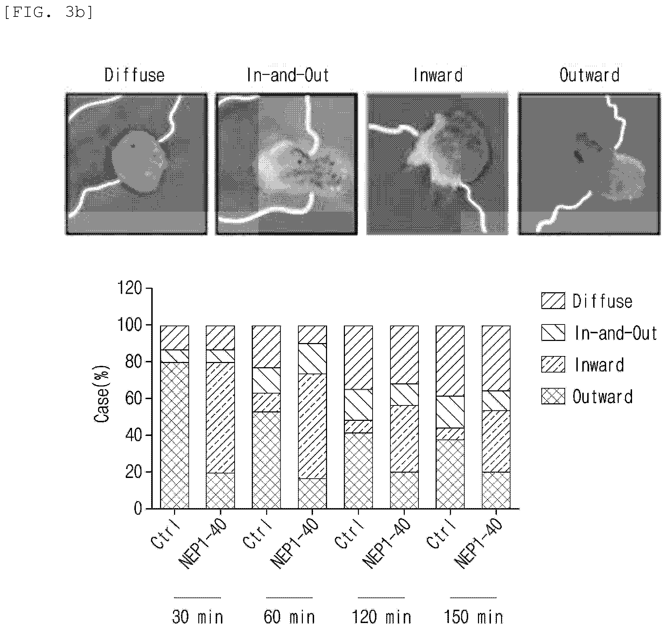

FIG. 3A illustrates the principle of measuring a density of actin formation when natural killer cells form an immunological synapse with the target cells, and FIG. 3B illustrates the results of confirming an actin formation behavior in the natural killer cells according to the inhibition of the activity of NgR1.

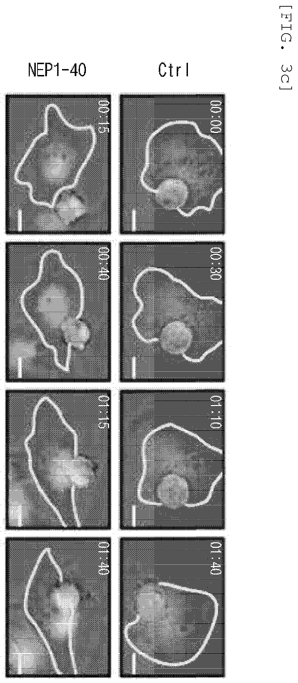

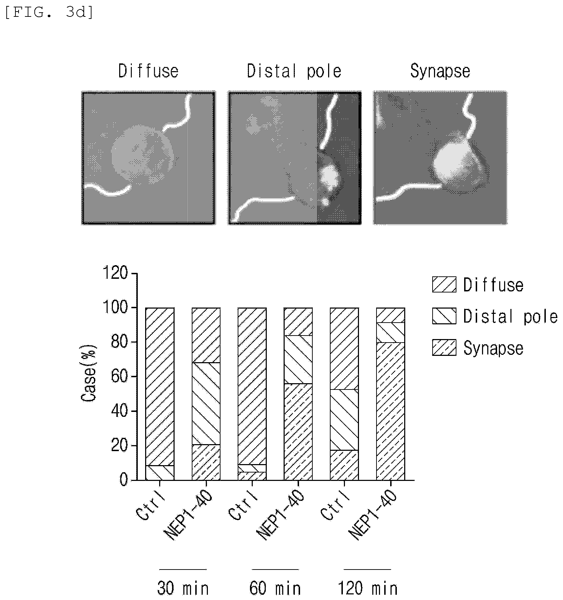

FIG. 3C illustrates a process of staining granules of the natural killer cells and measuring a granule formation process in real time over time using a fluorescence microscope, and FIG. 3D illustrates the results of confirming a granule formation behavior in the natural killer cells according to the inhibition of the activity of NgR1.

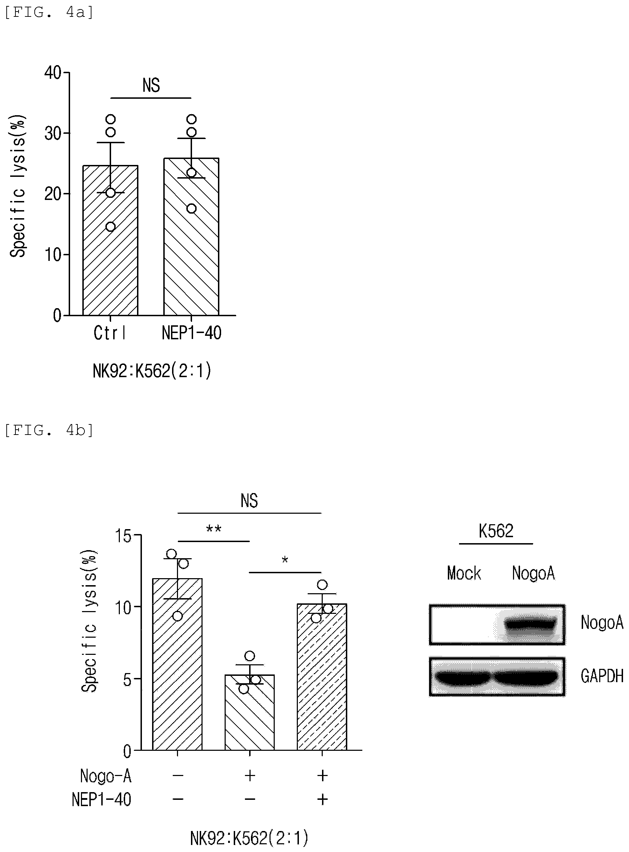

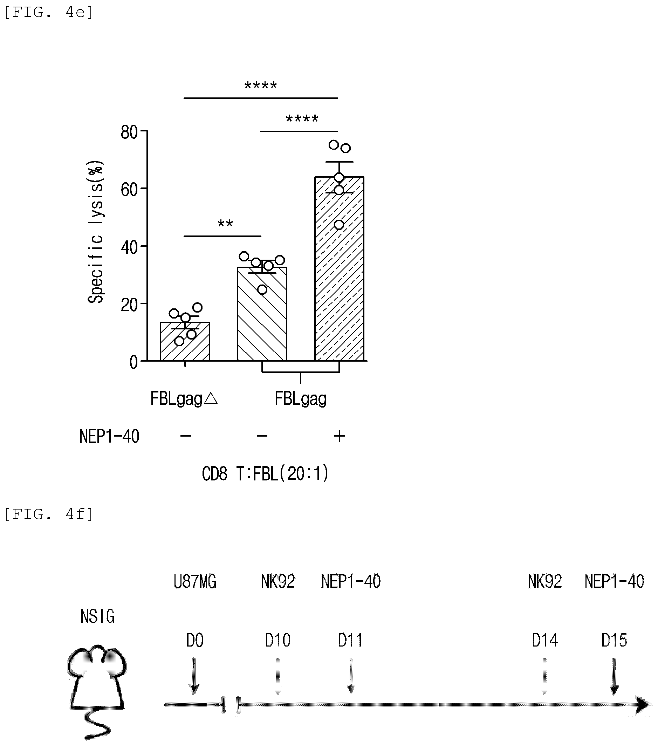

FIGS. 4A to 4D illustrate the results of in vitro confirmation that cytolytic activity or cytotoxicity of natural killer cells is reduced when NgR1 protein of the natural killer cells is activated, and FIG. 4E illustrates the results of in vitro confirmation that cytolytic activity or cytotoxicity of T cells is reduced when NgR1 protein of the T cells is activated.

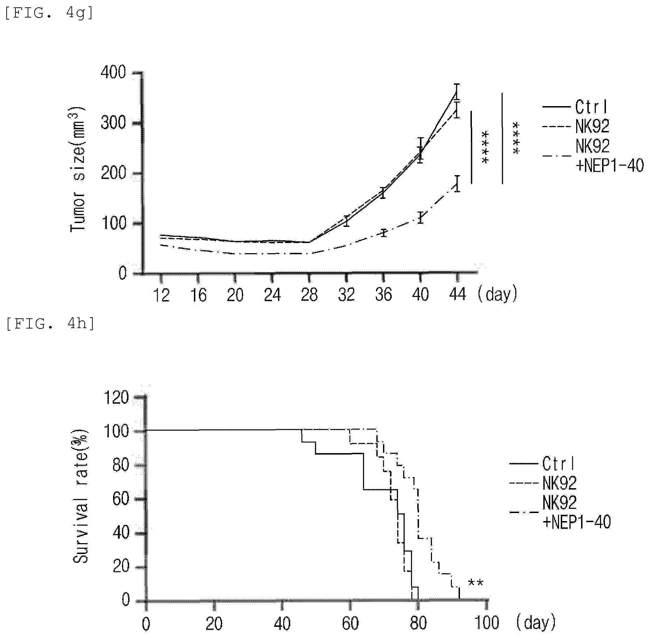

FIG. 4F illustrates a process of establishing an animal model by xenografting U87MG cell line expressing Nogo protein into a mouse, and FIGS. 4G and 4H illustrate the results of in vivo confirmation that cytolytic activity or cytotoxicity of natural killer cells is reduced when NgR1 protein of the natural killer cells is activated.

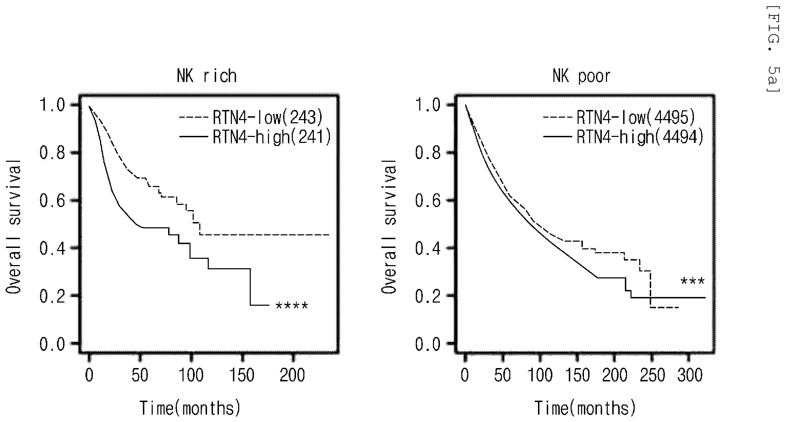

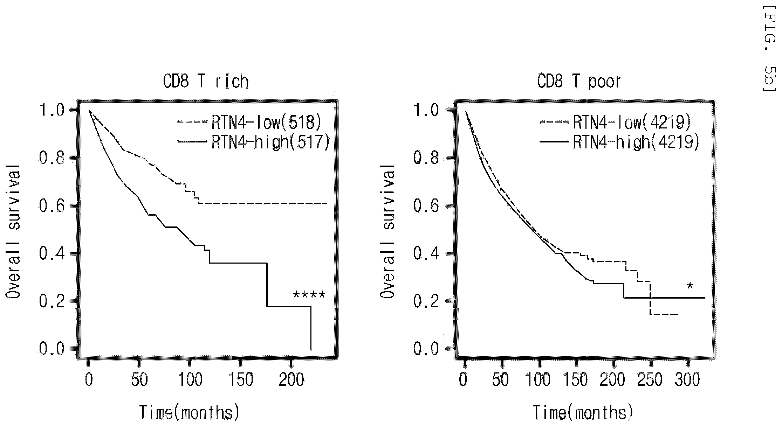

FIGS. 5A and 5B illustrate the results of diagnostic prognosis analysis using TCGA data to confirm an influence of a relationship between NgR1 of natural killer cells and T cells and Nogo protein of cancer cells on anticancer immunity, respectively.

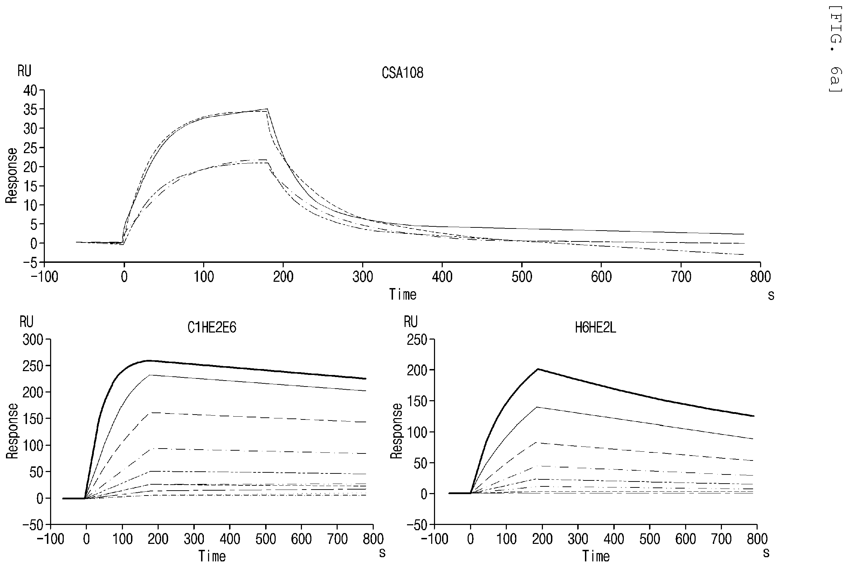

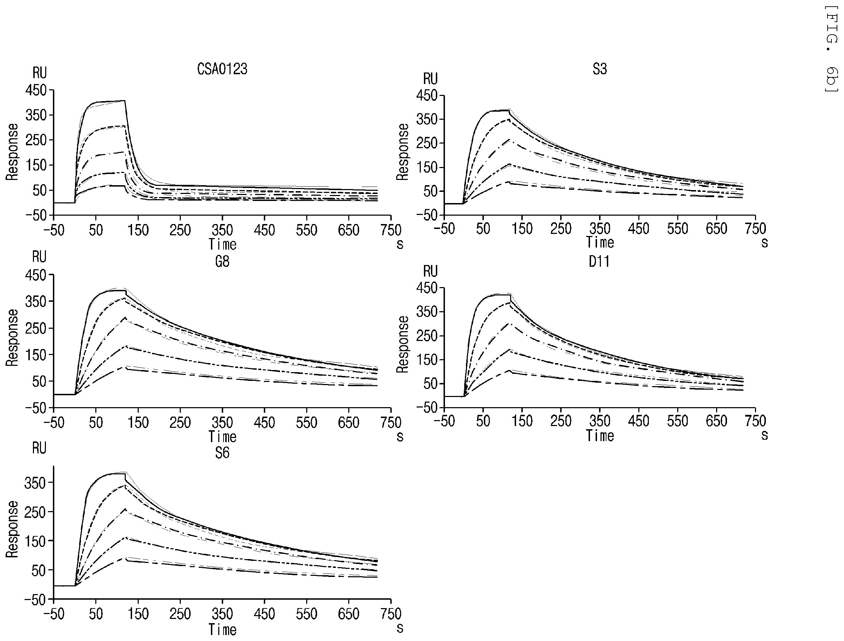

FIG. 6A illustrates the results of surface plasmon resonance analysis obtained by analyzing binding specificity of a novel CSA108 antibody and two types of anti-NgR1 antibodies obtained by optimizing the same to NgR1, and FIG. 6B illustrates the results of surface plasmon resonance analysis obtained by analyzing binding specificity of a novel CSA123 antibody and four types of anti-NgR1 antibodies obtained by optimizing the same to NgR1.

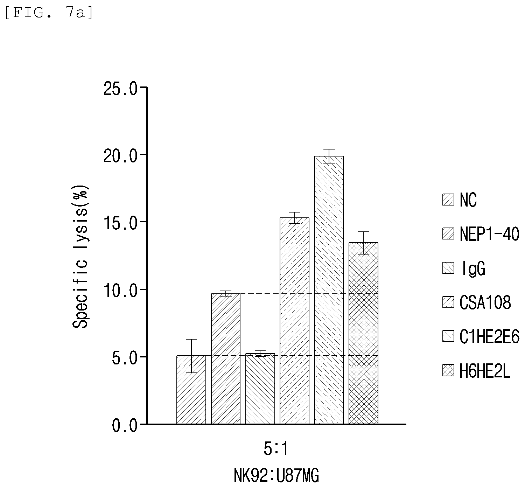

FIGS. 7A to 7C illustrate the results of in vitro confirmation that cytolytic activity or cytotoxicity of natural killer cells is enhanced when the activity of NgR1 is inhibited by the CSA108 antibody and two types of anti-NgR1 antibodies obtained by optimizing the same and the CSA123 antibody and four types of anti-NgR1 antibodies obtained by optimizing the same.

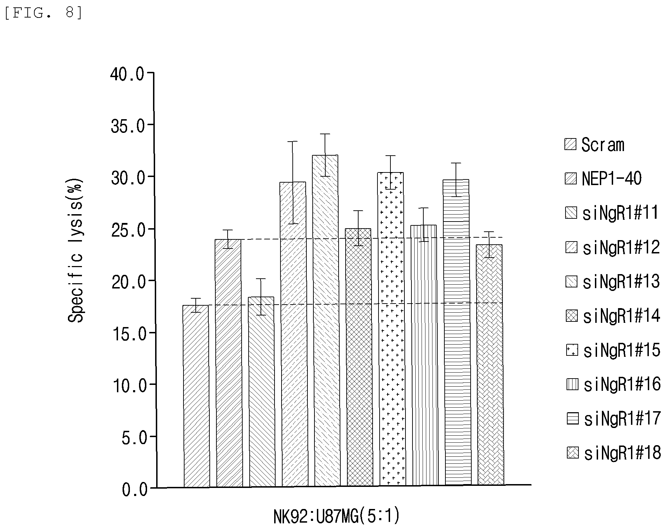

FIG. 8 illustrates the results of in vitro confirmation that cytolytic activity or cytotoxicity of natural killer cells is enhanced when the activity of NgR1 is inhibited by eight types of novel siRNAs (siNgR1 #11 to #18) that specifically bind to mRNA of NgR1.

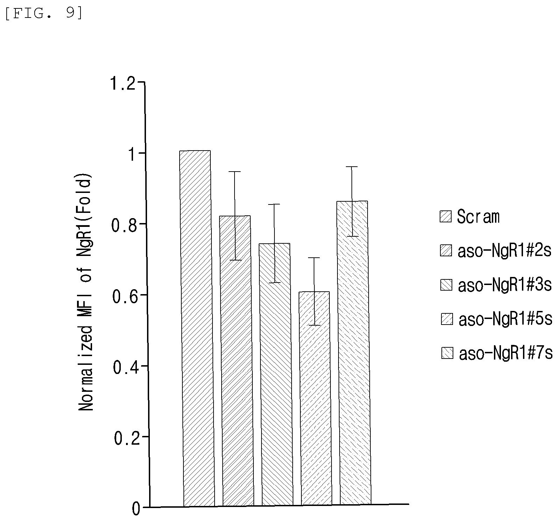

FIG. 9 illustrates the results of in vitro confirmation that the expression of NgR1 on an NK cell surface is reduced when NK cells are treated with four types of novel ASOs (aso-NgR1 #2s, #3s, #5s, and #7s) that specifically bind to mRNA of NgR1.

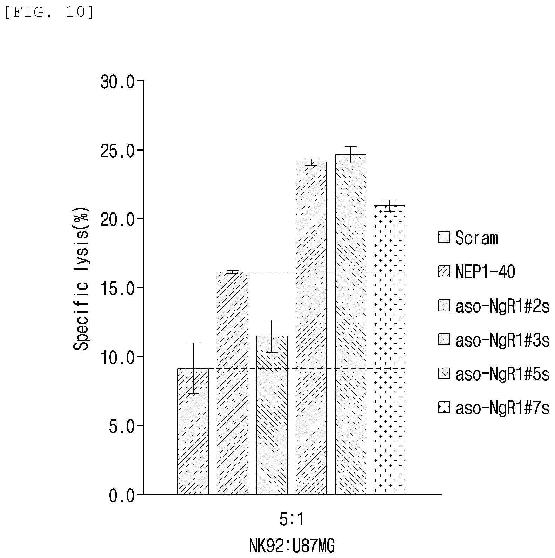

FIG. 10 illustrates the results of in vitro confirmation that cytolytic activity or cytotoxicity of natural killer cells is enhanced when the activity of NgR1 is inhibited by four types of novel ASOs (aso-NgR1 #2s, #3s, #5s, and #7s) that specifically bind to mRNA of NgR1.

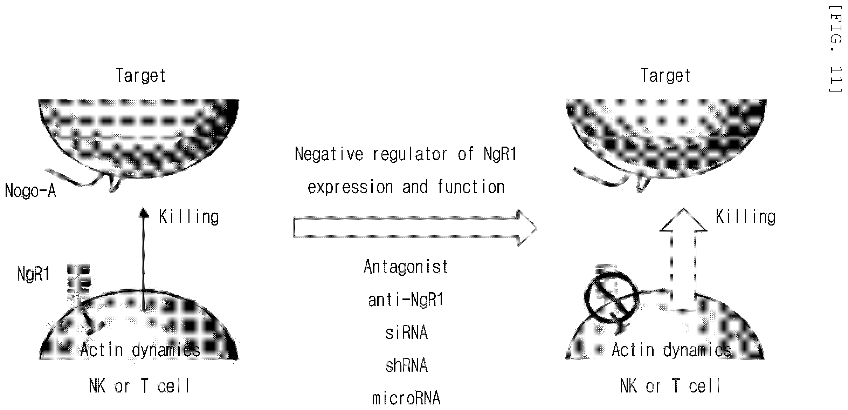

FIG. 11 is a schematic diagram illustrating the principle by which inhibition of activity of NgR1 in the present invention affects the activity of immune cells.

### BEST MODE FOR CARRYING OUT THE INVENTION

Hereinafter, the present invention will be described in detail.

### 1. Composition for Enhancing Cytolytic Activity by Promoting Formation of Immunological Synapse of Immune Cells

An aspect of the present invention provides a composition for enhancing cytolytic activity of immune cells.

The composition for enhancing cytolytic activity of immune cells may enhance the activity of the immune cells by promoting formation of an immunological synapse and/or regulating the immune cells, such that the cytolytic activity of target cells (for example, cancer cells and the like) of immune cells may be enhanced to inhibit the growth of the target cells and/or induce apoptosis of the target cells, thereby enhancing the cytolytic activity of the immune cells.

The composition for enhancing cytolytic activity of immune cells of the present invention contains an inhibitor for NgR1 protein as an active ingredient.

Specifically, the composition for enhancing cytolytic activity of immune cells of the present invention contains, as an active ingredient, an antibody or antigen-binding fragment thereof that specifically binds to NgR1 protein, or a small interfering RNA or an antisense oligonucleotide that specifically binds to NgR1 mRNA.

The composition for enhancing cytolytic activity of immune cells may further contain NEP1-40 that specifically binds to NgR1 protein as an active ingredient.

The immune cells may be cells capable of inducing immunity to kill target cells, and in particular, may be cells capable of killing target cells by lysing the target cells. For example, the immune cells may be natural killer cells (NK cells), T cells, natural killer T cells (NKT cells), cytokine induced killer cells (CIKs), macrophages, dendritic cells, and the like.

The NgR1 protein is a protein having a molecular weight of 50,708 Da and consisting of 473 amino acids, which is called "Nogo receptor 1", "Reticulon 4 receptor (RTN4R)", or "Nogo-66 Receptor (NgR)", and is a receptor that uses Nogo (or "Reticulon 4") protein, which is a neurite outgrowth inhibitor, as a ligand. An amino acid sequence of the NgR1 protein or a polynucleotide sequence of a gene encoding the same may be obtained from known databases (for example, GenBank No. NP_075380, NM_023004, and the like) such as NCBI's GeneBank. However, in addition to the above known sequences, a mutant in which some amino acids of the known amino acid sequence of NgR1 are mutated by a method such as addition, substitution, or deletion may also be included in the category of the NgR1 protein of the present invention.

In a specific embodiment of the present invention, it was confirmed that when the NgR1 protein was activated in immune cells such as natural killer cells or T cells, the inherent cytolytic activity or cytotoxicity of the immune cells against cancer cells was reduced, whereas the cytolytic activity or cytotoxicity of the immune cells against cancer cells was enhanced by inhibiting the activation of the NgR1 protein as described above, and it was confirmed from this that an inhibitor for NgR1 protein may be used as a means to enhance the cytolytic activity or cytotoxicity of immune cells. In particular, according to a specific embodiment of the present invention, it was confirmed that when the NgR1 protein was activated in natural killer cells, the formation of actin, which forms an immunological synapse in the initial stage where natural killer cells come into contact with their target cancer cells, was inhibited, and thus, the cytolytic activity or cytotoxicity of natural killer cells was ultimately negatively regulated. Therefore, it may be appreciated that the cytolytic activity or cytotoxicity of immune cells may be prevented from being reduced by the ligand of NgR1 protein by inhibiting the activation of NgR1 protein in immune cells such as natural killer cells or T cells or by inhibiting the expression of NgR1 protein itself.

The inhibitor for NgR1 protein refers to an agent that inhibits the activity or expression of NgR1 in immune cells, and may be, for example, an activity inhibitor of NgR1 protein that increases degradation of expressed NgR1 protein or interferes with its activity, or an expression inhibitor of NgR1 protein that reduces the expression of NgR1 protein at a transcription level.

The activity inhibitor of NgR1 protein may be a compound, peptide, peptide mimetic, aptamer, antibody, and the like that specifically bind to NgR1 protein.

The compound or peptide that specifically binds to the NgR1 protein may be obtained by a conventional method known in the technical field to which the present invention pertains, such as compound screening or phage display.

In the present invention, the peptide that specifically binds to the NgR1 protein may be NEP1-40, and the NEP1-40 may have an amino acid sequence of SEQ ID NO: 111, but is not limited thereto.

The peptide mimetic is a peptide or a non-peptide that inhibits a binding domain of the NgR1 protein, and may be composed of amino acids linked by non-peptide bonds such as psi bonds (Benkirane et al., J. Biol. Chem., 271: 33218-33224 (1996)). In addition, the peptide mimetic may be a novel small molecule that is structured similarly to the secondary structure characteristics of the NgR1 protein, may mimic the inhibitory properties of a large molecule such as an antibody or a water-soluble receptor (Park et al.,. Nat. Biotechnol., 18: 194-198 (2000); Takasaki et al., Nat. Biotechnol., 15: 1266-1270 (1997)), and may act with effects equivalent to a natural antagonist (Wrighton et al., Nat. Biotechnol., 15: 1261-1265 (1997)).

The aptamer refers to a single-stranded nucleic acid (DNA, RNA, or a modified nucleic acid) that has a stable tertiary structure in itself and has a characteristic of being able to bind to the NgR1 protein with high affinity and specificity. The aptamer is compared to a single antibody due to its characteristic of being able to bind to a target molecule with high affinity (usually pM level) and specificity, and in particular, has high potential as an alternative antibody to the point where it may be called a "chemical antibody". For the object of the present invention, the aptamer may be used without limitation as long as it may bind to the NgR1 protein and inhibit its activity.

The antibody, which is an immunoglobulin molecule and a multimer thereof having a structure in which one light chain is linked, by a disulfide bond, to each of two heavy chains linked to each other by a disulfide bond, refers to a substance capable of specifically binding to an antigenic site of the NgR1 protein, and may be produced by a conventional method known in the technical field to which the present invention pertains.

The light chain has two regions, that is, one variable region (light chain variable region (VL)) and one constant region (light chain constant region (CL)), and the heavy chain has four regions, that is, one variable region (heavy chain variable region (VH)) and three constant regions (heavy chain constant regions (CH), CH1, CH2, and CH3). The constant regions of the light chain and the heavy chain serve to impart biological properties such as binding between the light chains and/or the heavy chains, secretion, complement binding, and binding to an Fc receptor (FcR), and the variable regions of the light chain and the heavy chain determine recognition and binding specificity for an antigen. In particular, the binding specificity for the antibody is due to a structural complementarity between an antibody binding site and an epitope, and since the antibody binding site as described above is mainly composed of residues derived from three hypervariable regions called complementarity determining regions (CDRs) included in the variable regions of the heavy chain and the light chain, the complementarity determining regions as described above are referred to as amino acid sequences that define the binding specificity for the antibody. The three CDRs as described above are arranged between four relatively well-conserved regions called framework regions (FRs), which are arranged and linked in the order of FR1, CDR1, FR2, CDR2, FR3, CDR3, and FR4 in a direction from the N-terminus to the C-terminus. In this case, the three CDRs included in the heavy chain variable region are called CDR1-H, CDR2-H, and CDR3-H, respectively, and the three CDRs included in the light chain variable region are called CDR1-L, CDR2-L, and CDR3-L, respectively.

The "antigen-binding fragment" referred to in the present invention refers to a portion of an intact antibody, particularly, any polypeptide or glycoprotein including an antigen-binding site or a variable region of an intact antibody. The antigen-binding fragment as described above may be produced by a recombinant DNA technique or by enzymatically or chemically degrading the intact antibody, and examples of the antigen-binding fragment include, but are not limited to, Fv, Fab, F(ab')2, Fab', dsFv, (dsFv)2, scFv, sc(Fv)2, and diabodies, as well as bispecific and multispecific antibodies formed therefrom.

The Fab has a structure having variable regions of a light chain and a heavy chain, a constant region of the light chain, and a first constant region of the heavy chain (CH1 domain), and has one antigen-binding site. The Fab' differs from the Fab in that the Fab' has a hinge region containing one or more cysteine residues at the C-terminus of the heavy chain CH1 domain. The F(ab')2 is generated when the cysteine residue of the hinge region of the Fab' forms a disulfide bond. The Fv refers to the minimum antibody fragment having only a heavy chain variable region and a light chain variable region. The two-chain Fv is a fragment in which a heavy chain variable region and a light chain variable region are linked by a noncovalent bond, and the single-chain Fv is a fragment in which a heavy chain variable region and a light chain variable region are generally linked by a covalent bond via a peptide linker or are directly linked at the C-terminus, thereby forming a dimer-shaped structure like the two-chain Fv. The antigen-binding fragment may be prepared by using a protease (for example, the Fab may be obtained by restriction cleavage of the entire antibody with papain, and the F(ab')2 fragment may be obtained by cleavage with pepsin), or by a genetic recombination technique, but is not limited thereto.

The antibody of the present invention or the antigen-binding fragment thereof may further include, for example, a heavy chain constant region and/or a light chain constant region of a human-derived antibody, and the heavy chain constant region and/or the light chain constant region of the human-derived antibody may be used without limitation in type or amino acid sequence, as long as the antibody or the antigen-binding fragment thereof does not inhibit the property of specifically binding to NgR1.

The antibody of the present invention or the antigen-binding fragment thereof may include a heavy chain variable region containing CDR1-H having an amino acid sequence of SEQ ID NO: 1, SEQ ID NO: 41, SEQ ID NO: 43, or SEQ ID NO: 45, CDR2-H having an amino acid sequence of SEQ ID NO: 3, SEQ ID NO: 47, or SEQ ID NO: 49, and CDR3-H having an amino acid sequence of SEQ ID NO: 5, SEQ ID NO: 7, SEQ ID NO: 9, SEQ ID NO: 51, SEQ ID NO: 53, SEQ ID NO: 55, or SEQ ID NO: 57; and a light chain variable region containing CDR1-L having an amino acid sequence of SEQ ID NO: 23, SEQ ID NO: 79, or SEQ ID NO: 81, CDR2-L having an amino acid sequence of SEQ ID NO: 25, SEQ ID NO: 83, or SEQ ID NO: 85, and CDR3-L having an amino acid sequence of SEQ ID NO: 27, SEQ ID NO: 87, or SEQ ID NO: 89, or may have antigen specificity optimized based thereon, but is not particularly limited thereto.

In addition, the antibody of the present invention or the antigen-binding fragment thereof may include a heavy chain variable region having an amino acid sequence of SEQ ID NO: 11, SEQ ID NO: 13, SEQ ID NO: 15, SEQ ID NO: 59, SEQ ID NO: 61, SEQ ID NO: 63, SEQ ID NO: 65, or SEQ ID NO: 67; and a light chain variable region having an amino acid sequence of SEQ ID NO: 29, SEQ ID NO: 31, SEQ ID NO: 33, or SEQ ID NO: 91, may include a heavy chain having an amino acid sequence of SEQ ID NO: 17, SEQ ID NO: 19, SEQ ID NO: 21, SEQ ID NO: 69, SEQ ID NO: 71, SEQ ID NO: 73, SEQ ID NO: 75, or SEQ ID NO: 77, and a light chain having an amino acid sequence of SEQ ID NO: 35, SEQ ID NO: 37, SEQ ID NO: 39, or SEQ ID NO: 94, or may have antigen specificity optimized based thereon, but is not particularly limited thereto.

In an embodiment of the present invention, the antibody of the present invention or the antigen-binding fragment thereof may be a CSA108 antibody, and may be a C1HE2E6 antibody or H6HE2L antibody having antigen specificity optimized based thereon. In the present invention, the amino acid sequence of SEQ ID NO: 1 refers to CDR1-H sequences of the CSA108 antibody, C1HE2E6 antibody, and H6HE2L antibody, the amino acid sequence of SEQ ID NO: 3 refers to CDR2-H sequences of the CSA108 antibody, C1HE2E6 antibody, and H6HE2L antibody, the amino acid sequence of SEQ ID NO: 5 refers to a CDR3-H sequence of the CSA108 antibody, the amino acid sequence of SEQ ID NO: 7 refers to a CDR3-H sequence of the C1HE2E6 antibody, and the amino acid sequence of SEQ ID NO: 9 refers to a CDR3-H sequence of the H6HE2L antibody. The amino acid sequence of SEQ ID NO: 11 refers to a heavy chain variable region sequence of the CSA108 antibody, the amino acid sequence of SEQ ID NO: 13 refers to a heavy chain variable region sequence of the C1HE2E6 antibody, and the amino acid sequence of SEQ ID NO: 15 refers to a heavy chain variable region sequence of the H6HE2L antibody. The amino acid sequence of SEQ ID NO: 17 refers to the entire heavy chain sequence of the CSA108 antibody, the amino acid sequence of SEQ ID NO: 19 refers to the entire heavy chain sequence of the C1HE2E6 antibody, and the amino acid sequence of SEQ ID NO: 21 refers to the entire heavy chain sequence of the H6HE2L antibody.

In addition, in the present invention, the amino acid sequence of SEQ ID NO: 23 refers to CDR1-L sequences of the CSA108 antibody, C1HE2E6 antibody, and H6HE2L antibody, the amino acid sequence of SEQ ID NO: 25 (AAS) refers to CDR2-L sequences of the CSA108 antibody, C1HE2E6 antibody, and H6HE2L antibody, and the amino acid sequence of SEQ ID NO: 27 refers to CDR3-H sequences of the CSA108 antibody, C1HE2E6 antibody, and H6HE2L antibody. The amino acid sequence of SEQ ID NO: 29 refers to a light chain variable region sequence of the CSA108 antibody, the amino acid sequence of SEQ ID NO: 31 refers to a light chain variable region sequence of the C1HE2E6 antibody, and the amino acid sequence of SEQ ID NO: 33 refers to a light chain variable region sequence of the H6HE2L antibody. The amino acid sequence of SEQ ID NO: 35 refers to the entire light chain sequence of the CSA108 antibody, the amino acid sequence of SEQ ID NO: 37 refers to the entire light chain sequence of the C1HE2E6 antibody, and the amino acid sequence of SEQ ID NO: 39 refers to the entire light chain sequence of the H6HE2L antibody.

In another embodiment of the present invention, the antibody of the present invention or the antigen-binding fragment thereof may be a CSA123 antibody, and may be a G8 antibody, an S3 antibody, an S6 antibody, or a D11 antibody having antigen specificity optimized based thereon. In the present invention, the amino acid sequence of SEQ ID NO: 41 refers to CDR1-H sequences of the CSA123 antibody, S3 antibody, and D11 antibody, the amino acid sequence of SEQ ID NO: 43 refers to a CDR1-H sequence of the G8 antibody, the amino acid sequence of SEQ ID NO: 45 refers to a CDR1-H sequence of the S6 antibody, the amino acid sequence of SEQ ID NO: 47 refers to CDR2-H sequences of the CSA123 antibody and S3 antibody, the amino acid sequence of SEQ ID NO: 49 refers to CDR2-H sequences of the G8 antibody, S6 antibody, and D11 antibody, the amino acid sequence of SEQ ID NO: 51 refers to CDR3-H sequences of the CSA123 antibody and S6 antibody, the amino acid sequence of SEQ ID NO: 53 refers to a CDR3-H sequence of the G8 antibody, the amino acid sequence of SEQ ID NO: 55 refers to a CDR3-H sequence of the S3 antibody, and the amino acid sequence of SEQ ID NO: 57 refers to a CDR3-H sequence of the D11 antibody. The amino acid sequence of SEQ ID NO: 59 refers to a heavy chain variable region sequence of the CSA123 antibody, the amino acid sequence of SEQ ID NO: 61 refers to a heavy chain variable region sequence of the G8 antibody, the amino acid sequence of SEQ ID NO: 63 refers to a heavy chain variable region sequence of the S3 antibody, the amino acid sequence of SEQ ID NO: 65 refers to a heavy chain variable region sequence of the S6 antibody, and the amino acid sequence of SEQ ID NO: 67 refers to a heavy chain variable region sequence of the D11 antibody. The amino acid sequence of SEQ ID NO: 69 refers to the entire heavy chain sequence of the CSA123 antibody, the amino acid sequence of SEQ ID NO: 71 refers to the entire heavy chain sequence of the G8 antibody, the amino acid sequence of SEQ ID NO: 73 refers to the entire heavy chain sequence of the S3 antibody, the amino acid sequence of SEQ ID NO: 75 refers to the entire heavy chain sequence of the S6 antibody, and the amino acid sequence of SEQ ID NO: 77 refers to the entire heavy chain sequence of the D11 antibody.

In addition, in the present invention, the amino acid sequence of SEQ ID NO: 79 refers to CDR1-L sequences of the CSA123 antibody and D11 antibody, the amino acid sequence of SEQ ID NO: 81 refers to CDR1-L sequences of the G8 antibody, S3 antibody, and S6 antibody, the amino acid sequence of SEQ ID NO: 83 (AAS) refers to CDR2-L sequences of the CSA123 antibody and D11 antibody, the amino acid sequence of SEQ ID NO: 85 (YAA) refers to CDR2-L sequences of the G8 antibody, S3 antibody, and S6 antibody, the amino acid sequence of SEQ ID NO: 87 refers to CDR3-L sequences of the CSA123 antibody and D11 antibody, and the amino acid sequence of SEQ ID NO: 89 refers to CDR3-L sequences of the G8 antibody, S3 antibody, and S6 antibody. The amino acid sequence of SEQ ID NO: 91 refers to light chain variable region sequences of the CSA123 antibody, G8 antibody, S3 antibody, S6 antibody, and D11 antibody, and the amino acid sequence of SEQ ID NO: 94 refers to the entire light chain sequences of the CSA123 antibody, G8 antibody, S3 antibody, S6 antibody, and D11 antibody.

The amino acid sequences described above may include variants having different sequences due to deletion, insertion, or substitution of an amino acid residue, or a combination thereof within a range that does not affect a structure, function, activity, and the like of the polypeptide having the same. In addition, the amino acid sequences may include amino acids that have undergone common modifications known in the art, and examples of the modification of the amino acid include phosphorylation, sulfation, acrylation, glycosylation, methylation, and famesylation. The antibody of the present invention or the antigen-binding fragment thereof not only has the amino acid sequences described above, but also has an amino acid sequence that is substantially identical thereto or a variant thereof. The meaning of having the substantially identical amino acid sequence is that an amino acid sequence has 90% or more, 91% or more, 92% or more, 93% or more, 94% or more, 95% or more, 96% or more, 97% or more, 98% or more, 99% or more, or 99.5% or more homology with the amino acid sequence described above, but is not limited thereto.

The form of the antibody is not particularly limited, and a part of each of a polyclonal antibody, a monoclonal antibody, and an antibody having antigen binding ability is also included in the antibody of the present invention, and may include not only all immunoglobulin antibodies but also specific antibodies such as humanized antibodies. In addition, the antibody includes not only a complete form having two full-length light chains and two full-length heavy chains, but also a functional fragment of the antibody. The functional fragment of the antibody refers to a fragment having at least antigen binding ability, and may be Fab, F(ab'), F(ab')2, Fv, and the like. For the object of the present invention, the antibody may be used without limitation as long as it may bind to the NgR1 protein and inhibit its activity.

Another aspect of the present invention provides a gene encoding the entire or part of the antibody of the present invention or the antigen-binding fragment thereof.

In an embodiment of the present invention, a gene encoding each of CDRs included in the heavy chain variable region of the antibody of the present invention or the antigen-binding fragment thereof, that is, a gene encoding CDR1-H, a gene encoding CDR2-H, and a gene encoding CDR3-H, are each individually provided.

Specifically, the gene encoding each of CDRs included in the heavy chain variable region of the antibody of the present invention or the antigen-binding fragment thereof may encode each of CDRs included in the heavy chain variable regions of the CSA108 antibody of the present invention and the C1HE2E6 antibody and the H6HE2L antibody having antigen specificity optimized based thereon. More specifically, in the present invention, a base sequence of SEQ ID NO: 2 refers to a gene encoding CDR1-H of the CSA108 antibody, C1HE2E6 antibody, and H6HE2L antibody, a base sequence of SEQ ID NO: 4 refers to a gene encoding CDR2-H of the CSA108 antibody, C1HE2E6 antibody, and H6HE2L antibody, a base sequence of SEQ ID NO: 6 refers to a gene encoding CDR3-H of the CSA108 antibody, a base sequence of SEQ ID NO: 8 refers to a gene encoding CDR3-H of the C1HE2E6 antibody, and a base sequence of SEQ ID NO: 10 refers to a gene encoding CDR3-H of the H6HE2L antibody.

In addition, the gene encoding each of CDRs included in the heavy chain variable region of the antibody of the present invention or the antigen-binding fragment thereof may encode each of CDRs included in the heavy chain variable regions of the CSA123 antibody of the present invention and the G8 antibody, S3 antibody, S6 antibody, and D11 antibody having antigen specificity optimized based thereon. More specifically, in the present invention, a base sequence of SEQ ID NO: 42 refers to a gene encoding CDR1-H of the CSA123 antibody, S3 antibody, and D11 antibody, a base sequence of SEQ ID NO: 44 refers to a gene encoding CDR1-H of the G8 antibody, a base sequence of SEQ ID NO: 46 refers to a gene encoding CDR1-H of the S6 antibody, a base sequence of SEQ ID NO: 48 refers to a gene encoding CDR2-H of the CSA123 antibody and S3 antibody, a base sequence of SEQ ID NO: 50 refers to a gene encoding CDR2-H of the G8 antibody, S6 antibody, and D11 antibody, a base sequence of SEQ ID NO: 52 refers to a gene encoding CDR3-H of the CSA123 antibody and S6 antibody, a base sequence of SEQ ID NO: 54 refers to a gene encoding CDR3-H of the G8 antibody, a base sequence of SEQ ID NO: 56 refers to a gene encoding CDR3-H of the S3 antibody, and a base sequence of SEQ ID NO: 58 refers to a gene encoding CDR3-H of the D11 antibody.

In addition, in another embodiment of the present invention, each of a gene encoding a heavy chain variable region of the antibody of the present invention or the antigen-binding fragment thereof and a gene encoding the entire heavy chain of the antibody of the present invention or the antigen-binding fragment thereof is provided.

Specifically, the gene encoding the heavy chain variable region or the entire heavy chain of the antibody of the present invention or the antigen-binding fragment thereof may encode the heavy chain variable regions or the entire heavy chains of the CSA108 antibody of the present invention and the C1HE2E6 antibody and H6HE2L antibody having antigen specificity optimized based thereon. More specifically, in the present invention, a base sequence of SEQ ID NO: 12 refers to a gene encoding a heavy chain variable region of the CSA108 antibody, a base sequence of SEQ ID NO: 14 refers to a gene encoding a heavy chain variable region of the C1HE2E6 antibody, and a base sequence of SEQ ID NO: 16 refers to a gene encoding a heavy chain variable region of the H6HE2L antibody. A base sequence of SEQ ID NO: 18 refers to a gene encoding the entire heavy chain of the CSA108 antibody, a base sequence of SEQ ID NO: 20 refers to a gene encoding the entire heavy chain of the C1HE2E6 antibody, and a base sequence of SEQ ID NO: 22 refers to a gene encoding the entire heavy chain of the H6HE2L antibody.

In addition, the gene encoding the heavy chain variable region or the entire heavy chain of the antibody of the present invention or the antigen-binding fragment thereof may encode the heavy chain variable regions or the entire heavy chains of the CSA123 antibody of the present invention, and the G8 antibody, S3 antibody, S6 antibody, and D11 antibody having antigen specificity optimized based thereon. More specifically, in the present invention, a base sequence of SEQ ID NO: 60 refers to a gene encoding a heavy chain variable region of the CSA123 antibody, a base sequence of SEQ ID NO: 62 refers to a gene encoding a heavy chain variable region of the G8 antibody, a base sequence of SEQ ID NO: 64 refers to a gene encoding a heavy chain variable region of the S3 antibody, a base sequence of SEQ ID NO: 66 refers to a gene encoding a heavy chain variable region of the S6 antibody, and a base sequence of SEQ ID NO: 68 refers to a gene encoding a heavy chain variable region of the D11 antibody. A base sequence of SEQ ID NO: 70 refers to a gene encoding the entire heavy chain of the CSA123 antibody, a base sequence of SEQ ID NO: 72 refers to a gene encoding the entire heavy chain of the G8 antibody, a base sequence of SEQ ID NO: 74 refers to a gene encoding the entire heavy chain of the S3 antibody, a base sequence of SEQ ID NO: 76 refers to a gene encoding the entire heavy chain of the S6 antibody, and a base sequence of SEQ ID NO: 78 refers to a gene encoding the entire heavy chain of the D11 antibody.

In addition, in another embodiment of the present invention, a gene encoding each of CDRs included in the light chain variable region of the antibody of the present invention or the antigen-binding fragment thereof, that is, a gene encoding CDR1-L, a gene encoding CDR2-L, and a gene encoding CDR3-L, are each individually provided.

Specifically, the gene encoding each of CDRs included in the light chain variable region of the antibody of the present invention or the antigen-binding fragment thereof may encode each of CDRs included in the light chain variable regions of the CSA108 antibody of the present invention and the C1HE2E6 antibody and the H6HE2L antibody having antigen specificity optimized based thereon. More specifically, in the present invention, a base sequence of SEQ ID NO: 24 refers to a gene encoding CDR1-L of the CSA108 antibody, C1HE2E6 antibody, and H6HE2L antibody, a base sequence of SEQ ID NO: 26 (gct gca tcc) refers to a gene encoding CDR2-L of the CSA108 antibody, C1HE2E6 antibody, and H6HE2L antibody, and a base sequence of SEQ ID NO: 28 refers to a gene encoding CDR3-H of the CSA108 antibody, C1HE2E6 antibody, and H6HE2L antibody.

In addition, the gene encoding each of CDRs included in the light chain variable region of the antibody of the present invention or the antigen-binding fragment thereof may encode each of CDRs included in the light chain variable regions of the CSA123 antibody of the present invention and the G8 antibody, S3 antibody, S6 antibody, and D11 antibody having antigen specificity optimized based thereon. More specifically, in the present invention, a base sequence of SEQ ID NO: 80 refers to a gene encoding CDR1-L of the CSA123 antibody and D11 antibody, a base sequence of SEQ ID NO: 82 refers to a gene encoding CDR1-L of the G8 antibody, S3 antibody, and S6 antibody, a base sequence of SEQ ID NO: 84 (gct gca tcc) refers to a gene encoding CDR2-L of the CSA123 antibody and D11 antibody, a base sequence of SEQ ID NO: 86 (tat gct gca) refers to a gene encoding CDR2-L of the G8 antibody, S3 antibody, and S6 antibody, a base sequence of SEQ ID NO: 88 refers to a gene encoding CDR3-L of the CSA123 antibody and D11 antibody, and a base sequence of SEQ ID NO: 90 refers to a gene encoding CDR3-L of the G8 antibody, S3 antibody, and S6 antibody.

In addition, in another embodiment of the present invention, each of a gene encoding a light chain variable region of the antibody of the present invention or the antigen-binding fragment thereof and a gene encoding the entire light chain of the antibody of the present invention or the antigen-binding fragment thereof is provided.

Specifically, the gene encoding the light chain variable region or the entire light chain of the antibody of the present invention or the antigen-binding fragment thereof may encode the light chain variable regions or the entire light chains of the CSA108 antibody of the present invention and the C1HE2E6 antibody and H6HE2L antibody having antigen specificity optimized based thereon. More specifically, in the present invention, a base sequence of SEQ ID NO: 30 refers to a gene encoding a light chain variable region of the CSA108 antibody, a base sequence of SEQ ID NO: 32 refers to a gene encoding a light chain variable region of the C1HE2E6 antibody, and a base sequence of SEQ ID NO: 34 refers to a gene encoding a light chain variable region of the H6HE2L antibody. A base sequence of SEQ ID NO: 36 refers to a gene encoding the entire light chain of the CSA108 antibody, a base sequence of SEQ ID NO: 38 refers to a gene encoding the entire light chain of the C1HE2E6 antibody, and a base sequence of SEQ ID NO: 40 refers to a gene encoding the entire light chain of the H6HE2L antibody.

In addition, the gene encoding the light chain variable region or the entire light chain of the antibody of the present invention or the antigen-binding fragment thereof may encode the light chain variable regions or the entire light chains of the CSA123 antibody of the present invention, and the G8 antibody, S3 antibody, S6 antibody, and D11 antibody having antigen specificity optimized based thereon. More specifically, in the present invention, a base sequence of SEQ ID NO: 92 refers to a gene encoding light chain variable regions of the CSA123 antibody, G8 antibody, S3 antibody, and D11 antibody, and a base sequence of SEQ ID NO: 93 refers to a gene encoding a light chain variable region of the S6 antibody. A base sequence of SEQ ID NO: 95 refers to a gene encoding the entire light chains of the CSA123 antibody, G8 antibody, S3 antibody, and D11 antibody, and a base sequence of SEQ ID NO: 96 refers to a gene encoding the entire light chain of the S6 antibody.

The gene encoding the CDRs may undergo various modifications in the coding region within a range that does not change the amino acid sequences of the CDRs expressed from the coding region, and may undergo various mutations even in a portion excluding the coding region within a range that does not affect the expression of the gene. Finally, the gene for the CDRs may be mutated by substitution, deletion, or insertion of one or more nucleic acid bases, or a combination thereof, as long as it encodes a protein having equivalent activities, and such mutated genes are also included in the scope of the present invention.

In addition, the gene encoding the variable region of the heavy chain or the light chain may undergo various modifications in the coding region within a range that does not change the amino acid sequences of the variable regions expressed from the coding region, and may undergo various mutations even in a portion excluding the coding region within a range that does not affect the expression of the gene. Finally, the gene for the variable regions may be mutated by substitution, deletion, or insertion of one or more nucleic acid bases, or a combination thereof, as long as it encodes a protein having equivalent activities, and such mutated genes are also included in the scope of the present invention.

The gene of the present invention may have a base sequence substantially identical to the base sequences listed above. The substantially identical base sequence includes, for example, a case in which the same amino acid may be synthesized when transcribed and translated, and may be a base sequence having 90% or more, 91% or more, 92% or more, 93% or more, 94% or more, 95% or more, 96% or more, 97% or more, 98% or more, 99% or more, or 99.5% or more homology with the base sequences listed above, but is not limited thereto.

The gene encoding the antibody or antigen-binding fragment thereof may have an optimized base sequence depending on the type of organism into which the gene is to be introduced and expressed, and an expression system such as transcription or translation of the organism. This is due to degeneracy of a codon, and accordingly, various combinations of nucleotide sequences that may encode the protein expressed thereby may exist, all of which fall within the scope of the present invention. The modification of the polynucleotide according to the codon optimization may be determined according to the type of organism in which the antibody of the present invention or the antigen-binding fragment thereof is to be expressed and applied, and for example, the gene of the present invention may be a gene modified by being optimized for selection of a codon of a mammal or a primate, and more specifically, may be modified by being optimized to be suitable for the expression and action from a human.

In addition, still another aspect of the present invention provides a recombinant expression vector containing a gene encoding the entire or part of the antibody of the present invention or the antigen-binding fragment thereof, and a transformant into which the expression vector is introduced.

The recombinant expression vector refers to a genetic construct containing essential regulatory elements operably linked to an insert so that the insert is expressed within a cell, the recombinant expression vector of the present invention contains a gene encoding the entire or part of the antibody of the present invention or the antigen-binding fragment thereof, and the gene may be operably linked to a promoter.

The "operably linked" means that a region encoding a target protein is linked to a regulatory element in a manner that permits the expression of the nucleotide sequence.

The recombinant expression vector may be produced and purified using a standard recombinant DNA technique. The type of the recombinant expression vector is not particularly limited as long as it has a function of expressing a desired gene and producing a desired protein in various host cells of prokaryotic cells and eukaryotic cells, and may be a vector capable of producing a large amount of a target protein while having a promoter exhibiting strong activity and strong expression ability, but is not limited thereto. For example, the recombinant expression vector may be, but is not limited to, a vector composed of plasmids, cosmids, artificial chromosomes, liposomes, retrovirus, adenovirus, adenovirus-associated virus (AAV), vaccinia virus, herpes virus, lentivirus, or spumavirus.

In addition, the recombinant expression vector may further have an expression regulatory sequence such as a promoter, terminator, or enhancer, or a sequence for secretion, appropriately combined according to the purpose, depending on the type of host cell in which the entire or part of the antibody of the present invention or the antigen-binding fragment thereof is to be expressed or produced.

The recombinant expression vector may additionally contain a selection marker for selecting a host cell into which a vector is introduced, and may contain an origin of replication when it is a replicable expression vector.

In addition, the recombinant expression vector may have a sequence for facilitating purification of the expressed protein, and specifically, a gene encoding a tag for separation and purification may be linked to a gene encoding the entire or part of the antibody of the present invention or the antigen-binding fragment thereof so as to be operable. In this case, as the tag for separation and purification, GST, poly-Arg, FLAG, histidine-tag (His-tag), c-myc, or the like may be used alone, or two or more of them may be sequentially connected and used. A gene encoding the entire or part of the antibody of the present invention or the antigen-binding fragment thereof may be cloned through a restriction enzyme cleavage site, and in a case where a gene encoding a protease recognition site is used in the vector, it may be linked in-frame with a gene encoding the entire or part of the antibody of the present invention or the antigen-binding fragment thereof, such that when the entire or part of the antibody of the present invention or the antigen-binding fragment thereof is obtained and then cleaved with a protease, the entire or part of the antibody of the present invention or the antigen-binding fragment thereof in its original form may be produced.

In addition, a recombinant expression vector containing a gene encoding the entire or part of the antibody of the present invention or the antigen-binding fragment thereof is introduced into the transformant of the present invention.

A transformant may be produced by transforming the recombinant expression vector according to the present invention into any one appropriate host cell selected from the group consisting of bacteria, yeast, *E. coli,* fungi, plant cells, and animal cells, depending on the purpose of expression. For example, the host cell may be *E. coli (E. coli* BL21 (DE3), DH5α, or the like) or a yeast cell *(Saccharomyces, Pichia,* or the like). In this case, depending on the type of host cell, an appropriate culture method and medium conditions may be easily selected by those skilled in the art from known techniques in the field.

In addition, the present invention provides a method for producing an antibody or antigen-binding fragment thereof that specifically binds to NgR1 protein, the method including culturing the transformant.

As a method for introducing a recombinant expression vector for producing the transformant of the present invention, a known technique, that is, a heat shock method, an electric shock method, and the like may be used.

Since a protein expressed from the transformant is the antibody of the present invention or the antigen-binding fragment thereof, mass production of the antibody of the present invention or the antigen-binding fragment thereof may be facilitated by mass-cultivating the transformant and expressing the gene.

In the present invention, an expression inhibitor of the NgR1 protein may be a single-stranded or double-stranded nucleic acid sequence capable of causing RNA interference with mRNA of a gene encoding the NgR1 protein, and may be, for example, an antisense oligonucleotide, microRNA (miRNA), small interfering RNA (siRNA), short hairpin RNA (shRNA), or the like that complementarily binds to mRNA of the gene encoding the NgR1 protein. The single-stranded or double-stranded nucleic acid sequence capable of causing RNA interference with the mRNA of the gene encoding the NgR1 protein may be composed of a sequence having 70% or more, 75% or more, 80% or more, 85% or more, 90% or more, 95% or more, 99% or more, or 100% homology with a part of the mRNA of the gene encoding the NgR1 protein, and the sequence portion having the homology with a part of the mRNA of the gene encoding the NgR1 protein may be 7 bases or more, 10 bases or more, 13 bases or more, 15 bases or more, 18 bases or more, or 20 bases or more. In particular, the single-stranded or double-stranded nucleic acid sequence capable of causing the RNA interference with the mRNA of the gene encoding the NgR1 protein may include any one of the base sequences of SEQ ID NO: 41 to SEQ ID NO: 54, but is not particularly limited thereto. Meanwhile, the single-stranded or double-stranded nucleic acid sequence capable of causing the RNA interference with the mRNA of the gene encoding the NgR1 protein may be chemically or biochemically synthesized, or may be synthesized in vivo. For example, the single-stranded or double-stranded nucleic acid sequence capable of causing the RNA interference with the mRNA of the gene encoding the NgR1 protein may be synthesized in vitro using a method such as RNA polymerase I and administered into a living body, or may be synthesized in a living body using a vector in which the origin of the multi-cloning site (MCS) is in the opposite direction so that antisense RNA is transcribed.

The antisense oligonucleotide refers to DNA or RNA, or a derivative thereof, having a length of 6 to 100 bases, 8 to 60 bases, or 10 to 40 bases, containing a nucleic acid sequence complementary to the sequence of the mRNA of the gene encoding the NgR1 protein. The antisense oligonucleotide may bind to a complementary sequence in the mRNA of the gene encoding the NgR1 protein, and may thus act not only to inhibit the translation of the mRNA into a protein, but also to inhibit essential activities for the overall biological functions of the mRNA of the gene encoding the NgR1 protein, such as translocation into the cytoplasm or maturation.

The antisense oligonucleotide of the present invention may contain any one of base sequences of SEQ ID NO: 107 to SEQ ID NO: 110, and more specifically, may contain any one of the base sequences of SEQ ID NO: 108 to SEQ ID NO: 110, but is not particularly limited thereto.

The microRNA refers to DNA or RNA, or a derivative thereof, having a length of 10 to 40 bases, 12 to 30 bases, or 15 to 25 bases, containing a nucleic acid sequence complementary to a 3'-untranslated region (UTR) sequence of the mRNA of the gene encoding the NgR1 protein. The microRNA may complementarily bind to the 3'-UTR of the mRNA of the gene encoding the NgR1 protein, and may thus act not only to induce destabilization of the mRNA or inhibit the translation of the mRNA.

The small interfering RNA refers to a double-stranded RNA consisting of an RNA strand of a sense sequence having the same sequence as the mRNA of the gene encoding the NgR1 protein and an RNA strand of an antisense sequence having a sequence complementary thereto, and having a length of 10 to 40 bases, 12 to 30 bases, or 15 to 25 bases. The small interfering RNA may inhibit the expression of the NgR1 protein by complementarily binding to the mRNA of the gene encoding the NgR1 protein and inducing RNA interference phenomenon through cleavage of the mRNA.

The small interfering RNA of the present invention may have any one of base sequences of SEQ ID NO: 97 to SEQ ID NO: 106, specifically, may have any one of the base sequences of SEQ ID NO: 99 to SEQ ID NO: 106, and more specifically, may have any one of the base sequences of SEQ ID NO: 100 to SEQ ID NO: 106, but is not particularly limited thereto.

The short hairpin RNA refers to an RNA having a hairpin structure with a loop formed by intramolecular base pairing of a single-stranded RNA in which the sense sequence and the antisense sequence of the small interfering RNA for the mRNA of the gene encoding the NgR1 protein are linked by 3 to 10 nucleic acid sequences. The short hairpin RNA may be converted into siRNA by a dicer in cells and may cause RNA interference.

The base sequences described above may include variants having different sequences due to deletion, insertion, or substitution of a nucleic acid base, or a combination thereof within a range that does not affect a structure, function, activity, and the like of the polypeptide having the same. In addition, the base sequences may include nucleotides that have undergone a conventional modification known in the art, and the modification may include a modification of a base, a modification of a pentose, a modification of a phosphate, a modification of a linkage between bases, or any combination thereof. The small interfering RNA or the antisense oligonucleotide of the present invention not only has the base sequences described above, but also has a base sequence that is substantially identical thereto or a variant thereof. The substantially identical base sequence includes, for example, a case in which the same amino acid may be synthesized when transcribed and translated, and may be a base sequence having 90% or more, 91% or more, 92% or more, 93% or more, 94% or more, 95% or more, 96% or more, 97% or more, 98% or more, 99% or more, or 99.5% or more homology with the base sequences listed above, but is not limited thereto.

Meanwhile, since immune cells such as natural killer cells and T cells basically exhibit cytolytic activity or cytotoxicity against cancer cells, formation of an immunological synapse may be promoted through an inhibitor for the NgR1 protein as described above, and the cytolytic activity or cytotoxicity of the immune cells themselves may be enhanced.

Therefore, the composition for enhancing cytolytic activity of immune cells of the present invention may be used for prevention or treatment of an NgR1-associated disease.

The NgR1-associated disease may be one or more selected from the group consisting of cancer, multiple sclerosis, ALS, Huntington's disease, Alzheimer's disease, Parkinson's disease, diabetic neuropathy, stroke, traumatic brain injury, and spinal cord injury, but is not limited thereto, and may include any NgR1-associated disease.

The cancer may be one or more selected from the group consisting of skin cancer, breast cancer, uterine cancer, esophageal cancer, stomach cancer, brain tumor, glioblastoma, colon cancer, rectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, blood cancer, thymus cancer, urethral cancer, and bronchial cancer, but is not limited thereto.

In particular, in the cancer, a ligand that activates the NgR1 protein of immune cells may be expressed, particularly overexpressed. The ligand for the NgR1 protein may be Nogo (or "Reticulon 4") protein, which is a neurite outgrowth inhibitor, or may be an isoform of Nogo protein such as NogoA, Nogo-B, or Nogo-C, or a part of a domain thereof (for example, Nogo-66). In the case of the cancer expressing or overexpressing a ligand for the NgR1 protein as described above, the NgR1 protein of the immune cell is activated to reduce the cytolytic activity or cytotoxicity of the immune cells themselves, but the activation of the NgR1 protein of the immune cells is inhibited through the inhibitor of the NgR1 protein of the present invention, such that the reduction in the cytolytic activity or cytotoxicity of the immune cells themselves as described above may be prevented.

The composition may further contain NEP1-40, which is an inhibitor of NgR1 protein activity.

Meanwhile, the composition for enhancing cytolytic activity of immune cells of the present invention may be an anticancer adjuvant. The anticancer adjuvant refers to a composition that may enhance the effect of anticancer treatment by activating the immune function of the immune cells, and the anticancer treatment may be radiation treatment, anticancer agent treatment, or immuno-oncology agent treatment.

The compositions of the present invention may additionally contain an immuno-oncology agent in addition to the active ingredients described above.

The immuno-oncology agent may be one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-CD73, anti-ICOS, anti-CD40L, anti-BTLA, anti-TCR, anti-TIGIT, anti-VISTA, and anti-NKG2A, but is not limited thereto.

In still another aspect, the present invention provides a chimeric antigen receptor (CAR) containing the antibody of the present invention or the antigen-binding fragment thereof.

The term "chimeric antigen receptor" or "CAR" refers to an engineered receptor containing an extracellular antigen-binding domain and an intracellular signaling domain. Although the most common type of CAR contains a single chain variable fragment (scFv) derived from a monoclonal antibody fused to transmembrane and intracellular domains of a T cell coreceptor, such as a CD3ζ chain, the present invention described herein is not limited to these domains. Rather, the "chimeric antigen receptor" or "CAR" as used herein refers to any receptor engineered to express any intracellular signaling molecule and an extracellular antigen-binding domain fused or linked thereto.

In the present invention, the binding domain may include an antigen-binding fragment provided in the present invention, and preferably scFv.

The chimeric antigen receptor of the present invention may further include at least one of a hinge region (or a spacer) and a signaling domain.

In the present invention, the hinge region is a region connecting the antigen-binding domain and the transmembrane domain, is also called a "spacer", and has the purpose of extending the antigen-binding domain from the T cell membrane or NK cell membrane.

In the present invention, the hinge region may be obtained from any suitable sequence from any genus, including, for example, a human or a part thereof, or may include a hinge region of a human protein containing CD8, CD28, 4-1BB, OX40, the entire or part of a CD3 zeta (ζ) chain, a T cell receptor α or β chain, CD28, CD3ε, CD45, CD4, CD5, CD8, CD9, CD16, CD22, CD33, CD37, CD64, CD80, CD86, CD134, CD137, ICOS, CD154, a functional derivative thereof, or a combination thereof commonly used in the art, but is not limited thereto.

In addition, in the present invention, the hinge region may include one selected from immunoglobulins (for example, IgG1, IgG2, IgG3, IgG4, and IgD) without being limited to immunoglobulins, but is not limited thereto.

In the present invention, the signaling domain refers to a portion of the chimeric antigen receptor that is found or is engineered to be found inside a T cell or N cell. In the present invention, the signaling domain may or may not include a transmembrane domain that functions to anchor the chimeric antigen receptor in a plasma membrane of a T cell or N cell.

In the present invention, the transmembrane domain and the signaling domain may be derived from the same protein (for example, Cd3ζ), and alternatively, the transmembrane domain and the signaling domain may be derived from different proteins (for example, a transmembrane domain of CD28 and an intracellular signaling domain of a CD3ζ molecule, or vice versa) .

In the present invention, the transmembrane domain contains a hydrophobic polypeptide that spans the cell membrane. In particular, the transmembrane domain may span from one side of a cell membrane (extracellular) through the other side of the cell membrane (intracellular or cytoplasmic).

In the present invention, the transmembrane domain may be in the form of an alpha helix or a beta barrel, or a combination thereof. In addition, in the present invention, the transmembrane domain may contain a polytopic protein having many transmembrane segments, each alpha-helical, beta sheets, or a combination thereof.

In the present invention, the transmembrane domain may contain, for example, a T cell receptor α or β chain, the entire or part of a CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, a functional derivative thereof, or a combination thereof, but is not limited thereto.

In the present invention, the transmembrane domain may be, for example, a polypeptide containing hydrophobic residues such as leucine and valine, which is artificially designed. In an embodiment of the present invention, a triplet of phenylalanine, tryptophan, and valine may be found at each end of the synthetic transmembrane domain.

In the present invention, at least one costimulatory domain may be further included between the transmembrane domain and the signaling domain. In the present invention, the costimulatory domain is a portion through which a costimulatory signal is transmitted, and may serve to help CAR-T cells or CAR-NK cells that recognize a specific antigen bound to the antigen-binding domain to self-proliferate while eliciting an immune response, and act to transmit a signal so as to increase the in vivo retention time of the cells.

In the present invention, the costimulatory domain may include, but is not limited to, a functional signaling domain derived from a polypeptide containing 4-1BB (CD137); OX40; CD27; CD28; CD30; CD40; PD-1; CD2; CD7; CD258; natural killer group 2 member C (NKG2C); natural killer group 2 member (NKG2D); B7-H3; CD83; ICAM-1; a ligand that binds to LFA-1 (CD11a/CD18) or ICOS; an active fragment thereof; a functional inducer thereof; or a combination thereof.

In the present invention, the signaling domain may contain a polypeptide that provides activation of immune cells to stimulate or activate at least a part of a surface of an immune cell signaling pathway.

In the present invention, the signaling domain may include, but is not limited to, a functional signaling domain derived from a polypeptide containing the entire or part of CD3 zeta (ζ), common FcR gamma (FcER1G), Fc gamma RIIIa, FcR beta (Fc epsilon rib), CD3 gamma, CD3 delta, CD3 epsilon, CD79a, CD79b, DNAX-activating protein 10 (DAP10), DNAX-activating protein 12 (DAP12), an active fragment thereof, a functional derivative thereof, or a combination thereof, and such signaling domains are known in the art.

In addition, the chimeric antigen receptor of the present invention may further contain a signal peptide.

In the present invention, the "signal peptide" has a peptide sequence, which is a kind of peptide of any secreted or transmembrane protein, may direct the transport of the chimeric antigen receptor of the present invention to the cell membrane and cell surface, and may provide correct localization of the chimeric antigen receptor of the present invention. In particular, the signal peptide in the present invention supports the chimeric antigen receptor of the present invention to the cell membrane, such that the extracellular portion of the chimeric antigen receptor is displayed on the cell surface, the transmembrane portion spans the plasma membrane, and the active domain is in the cytoplasmic portion, or interior of the cell.

In addition, the pharmaceutical compositions of the present invention may further contain a pharmaceutically acceptable carrier or additive in addition to the active ingredients as described above.

The meaning of "pharmaceutically acceptable" means that it does not inhibit the activity of the active ingredient and does not have toxicity exceeding the adaptability of the subject of application (prescription). The "carrier" is defined as a compound that allows a compound to be easily incorporated into cells or tissue.

The active ingredients of the present invention may be administered alone or in combination with any convenient carrier and the like, and a formulation to be administered may be a single-dose or multiple-dose formulation. The pharmaceutical composition may be a solid formulation or a liquid formulation. The solid formulation includes, but is not limited to, a powder, a granule, a tablet, a capsule, a suppository, and the like. The solid formulation may include, but is not limited to, a carrier, a flavoring agent, a binder, a preservative, a disintegrant, a lubricant, a filler, and the like. The liquid formulation includes water, a solution such as a propylene glycol solution, a suspension, an emulsion, and the like, but is not limited thereto, and may be prepared by adding an appropriate colorant, flavoring agent, stabilizer, thickener, and the like. For example, a powder may be prepared by simply mixing a tetra-hydroxy derivative of a polysaturated fatty acid, which is an active ingredient of the present invention, with an appropriate pharmaceutically acceptable carrier such as lactose, starch, or microcrystalline cellulose. A granule may be prepared by mixing the active ingredients of the present invention, an appropriate pharmaceutically acceptable carrier, and an appropriate pharmaceutically acceptable binder such as polyvinylpyrrolidone or hydroxypropyl cellulose, and then utilizing a wet granulation method using a solvent such as water, ethanol, or isopropanol, or a dry granulation method using a compressive force. In addition, a tablet may be prepared by mixing the granule with an appropriate pharmaceutically acceptable lubricant such as magnesium stearate, and then tableting the mixture using a tableting machine.

The active ingredients of the present invention may be administered in the form of an oral agent, an injection (for example, an intramuscular injection, an intraperitoneal injection, an intravenous injection, an infusion, a subcutaneous injection, or an implant), an inhalation agent, a nasal administration agent, a vaginal agent, a rectal administration agent, a sublingual agent, a transdermal agent, a topical agent, or the like, depending on a disease to be treated and a condition of an individual, but are not limited thereto. Depending on a route of administration, the pharmaceutical composition may be formulated into an appropriate dose unit formulation containing a pharmaceutically acceptable carrier, additive, and vehicle, which are commonly used and non-toxic.

The pharmaceutical composition of the present invention may be administered at a daily dosage of about 0.0001 mg/kg to about 10 g/kg, and may be administered at a daily dosage of about 0.001 mg/kg to about 1 g/kg. However, the dosage may vary depending on a degree of purification of the mixture, a patient's condition (age, gender, weight, or the like), and severity of the condition being treated. If necessary, for convenience, the total daily dosage may be administered in divided doses several times throughout a day.

### 2. Method for Diagnosing Cancer Patient and Method for Screening Anticancer Agent

Still another aspect of the present invention provides a method for diagnosing prognosis of a cancer patient or providing information for determining a treatment strategy.

The method for diagnosing prognosis of a cancer patient or providing information for determining a treatment strategy of the present invention includes measuring an expression level of Nogo protein in cancer cells isolated from the cancer patient, and comparing the expression level with an expression level of Nogo protein in normal cells.

The measuring of the expression level of the Nogo protein may be measuring the amount of mRNA transcribed from a gene encoding the NgR1 protein or the amount of protein translated from the mRNA.

The measuring of the amount of mRNA transcribed from the gene encoding the Nogo protein may be performed using reverse transcription-polymerase chain reaction (RT-PCR), competitive RT-PCR, real-time RT-PCR, RNase protection assay (RPA), Northern blotting, DNA chips, and the like, and in this case, a primer pair, a probe, or an antisense oligonucleotide for the mRNA may be used.

In addition, the measuring of the amount of Nogo protein may be performed using enzyme-linked immunosorbent assay (ELISA), immunohistochemistry, Western blotting, flow cytometry (FACS), radioimmunoassay (RIA), radioimmunodiffusion, tissue immunostaining, immunoprecipitation assay, protein chips, and the like, and in this case, a peptide, a peptide mimetic, an aptamer, an antibody, and the like for the protein may be used.

Meanwhile, the Nogo protein expressed in the cancer cells activates the NgR1 protein of immune cells, thereby reducing the cytolytic activity or cytotoxicity of the immune cells; thus, in the case of cancer cells in which the expression level of the Nogo protein is higher than that of normal cells, anticancer treatment using immune cells may not be effective. Therefore, the method for diagnosing prognosis of a cancer patient or providing information for determining a treatment strategy of the present invention may further include determining that anticancer treatment using immune cells is not effective when the expression level of the Nogo protein measured in the cancer cells is higher than the expression level of the Nogo protein in normal cells, or determining that anticancer treatment using immune cells is effective when the expression level of the Nogo protein measured is not higher than the expression level of the Nogo protein in normal cells. For example, a case where the expression level of the Nogo protein measured in cancer cells is higher than the expression level of the Nogo protein in normal cells may be determined that the prognosis may not be good even when an anticancer agent using immune cells is administered, and a treatment strategy may be determined to use a different type of anticancer agent rather than an anticancer agent using immune cells. In addition, when the expression level of the Nogo protein measured in cancer cells is not higher than the expression level of the Nogo protein in normal cells, a treatment strategy may be determined to actively use an anticancer agent using immune cells. Therefore, the prognosis may be a prognosis after administration of an anticancer agent using immune cells, and the treatment strategy may be a strategy of utilizing an anticancer agent using immune cells.

The case where the expression level of the Nogo protein measured in cancer cells isolated from the cancer patient is higher than that in normal cells may be a case where the expression level of the Nogo protein measured in cancer cells isolated from the cancer patient is higher than that in normal cells by 1% or more, 2% or more, 3% or more, 4% or more, 5% or more, 10% or more, 20% or more, 30% or more, 40% or more, 50% or more, 60% or more, 70% or more, 80% or more, 90% or more, or 100% or more.

Meanwhile, still another aspect of the present invention provides a method for screening a novel anticancer agent.

The method for screening an anticancer agent of the present invention includes treating a candidate substance to immune cells; measuring an expression level or activity level of NgR1 protein in the immune cells treated with the candidate substance; and comparing the measured expression level or activity level of the NgR1 protein with an expression level or activity level of NgR1 protein in immune cells not treated with the candidate substance.

The candidate substance is a newly synthesized or known compound, and refers to a substance expected to inhibit the expression or activity of the NgR1 protein.

In addition, the measuring of the expression or activity level of the NgR1 protein may be measuring the amount of mRNA transcribed from a gene encoding the NgR1 protein or the amount of protein translated from the mRNA, and specific means for this are as described above.

In addition, the method for screening an anticancer agent may further include comparing the measured expression or activity level of the NgR1 protein with that before treating the candidate substance, and determining the candidate substance as an anticancer agent when the expression or activity level of the NgR1 protein measured after treating the candidate substance is reduced compared to that before treating the candidate substance. The case where the expression or activity level of the NgR1 protein measured after treating the candidate substance is reduced compared to that before treating the candidate substance may be a case where the expression or activity level of the NgR1 protein measured after treating the candidate substance is reduced by 1% or more, 2% or more, 3% or more, 4% or more, 5% or more, 10% or more, 20% or more, 30% or more, 40% or more, 50% or more, 60% or more, 70% or more, 80% or more, 90% or more, or 100% or more, compared to before treating with the candidate substance.

Still another aspect of the present invention provides a method for enhancing cytolytic activity of immune cells, the method including administering the composition for enhancing cytolytic activity of immune cells to an individual.

Still another aspect of the present invention provides a method for preventing or treating cancer, the method including administering the composition for enhancing cytolytic activity of immune cells to an individual.

Still another aspect of the present invention provides use of the composition for enhancing cytolytic activity of immune cells for cytolytic activity of immune cells.

Still another aspect of the present invention provides use of the composition for enhancing cytolytic activity of immune cells for preventing or treating cancer.

Hereinafter, the present invention will be described in detail with reference to Examples.

However, the following Examples are intended to specifically illustrate the present invention, and the present invention is not limited by the following Examples.

### [Example 1]

### Confirmation of Expression of NgR1 in Immune cells and Signaling by Expression

First, it was confirmed whether NgR1 was expressed even in natural killer cells (NK cells) and T cells. Specifically, a fluorescent dye was conjugated to an antibody that specifically binds to an extracellular portion of NgR1 protein, or surfaces of NK cells and T cells were stained using an antibody conjugated with a fluorescent dye, and the expression of NgR1 in the NK cells and T cells was confirmed using flow cytometry.

As a result, as illustrated in FIG. 1A, it was confirmed that NgR1 was expressed on the cell surfaces of all human cord blood NK cells (UCB-NK), differentiated mature NK cells (mNK), and NK92 cells. In addition, as illustrated in FIG. 1B, it was confirmed that NgR1 was expressed on the cell surfaces of all human cord blood CD8 T cells (UCB-CD8) and Jurkat cells.

In addition, NgR1 was activated by treating NK92 cells with Nogo-P4 peptide, which is a ligand of NgR1, and the expression of molecules known as downstream signals of NgR1 was confirmed. Specifically, in order to maintain the survival of NK92 cells, NK92 cells were cultured for 24 hours in a medium in which IL-2, which is a cytokine added to the medium, was removed, and then treated with Nogo-P4 peptide for 0 minutes, 10 minutes, 30 minutes, and 60 minutes to activate NgR1 in each NK92 cell. As described above, NK92 cells treated with Nogo-P4 peptide for 0 minutes, 10 minutes, 30 minutes, and 60 minutes were lysed, proteins were isolated from the cell lysates, and the expression of molecules known as downstream signals of NgR1 was confirmed by Western blotting.

As a result, as illustrated in FIG. 1C, it was also confirmed that intracellular downstream signaling was activated by activation of NgR1 even in NK cells.

### [Example 2]

### Confirmation of step in Which NgR1 Is Involved During Contact Process between Natural Killer Cells and Target Cells (Cancer Cells)

It was confirmed at which step NgR1 is involved in the mechanism by which NK cells exhibit cytolytic activity. The inventors of the present invention assumed that NgR1 affects the "contact" process between NK cells and target cells (cancer cells), and to confirm this, the inventors first established the contact process between NK cells and target cells into three steps as illustrated in FIG. 2A.

In order to determine which of the steps established as above is changed by the inhibition of NgR1, target cells (U87MG cells) and NK cells were co-cultured in the presence or absence of NEP1-40, which is a (competitive) inhibitor for NgR1, and each step was analyzed in real time using a microscope. The transient contact step and the contact duration were able to be analyzed by analyzing individual single NK cells in real time.

As a result, as illustrated in FIGS. 2B and 2C, it was confirmed that, when the activity of NgR1 was inhibited by treating with NEP1-40, the proportion of the transient contact step in the contact process between NK cells and target cells (U87MG cells) was decreased (FIG. 2B), and the contact duration was increased (FIG. 2C).

### [Example 3]

### Confirmation of Negative Role of NgR1 in Immunological Synapse Formation Process

Based on the above results, the inventors of the present invention thought that NgR1 affects the formation of an immunological synapse between NK cells and target cells (cancer cells), and to confirm this, the inventors first produced an NK cell line (lifeact-NK92) that continuously expresses GFP-conjugated actin. The lifeact-NK92 was co-cultured with target cells, and as illustrated in FIG. 3A, a formation density of actin exhibiting fluorescence in lifeact-NK92 during contact of lifeact-NK92 to target cells was measured in real time using a fluorescence microscope. Through this, it was able to confirm whether actin exhibiting fluorescence was spread throughout the entire cell, or formed either toward or away from the target cell, toward the target cell, or away from the target cell, according to the inhibition of NgR1.

As a result, as illustrated in FIG. 3B, it was confirmed that as the activity of NgR1 was inhibited, the actin of the NK cells rapidly became denser toward the target cell.

In addition, in the process of forming an immunological synapse between NK cells and target cells (cancer cells), it was confirmed whether the NgR1 affects the granule polarization of NK cells. To this end, the granules of the NK cells were stained with LysoSensor^{™} and co-cultured with target cells in the presence or absence of NEP1-40, which is a (competitive) inhibitor for NgR1, and the polarization process of granules exhibiting fluorescence in the NK cells during the contact of the stained granules with the target cells was measured in real time using a fluorescence microscope, as illustrated in FIG. 3C. Through this, it was able to confirm whether the granules exhibiting fluorescence were spread throughout the entire cell, or polarized toward the target cell or away from the target cell, according to the inhibition of NgR1.

As a result, as illustrated in FIG. 3D, it was confirmed that when the activity of NgR1 was inhibited by treating with NEP1-40, the granules of the NK cells rapidly became denser toward the target cell.

### [Example 4]

### Confirmation of Effect of Activation of NgR1 on Cytolytic Activity of Immune Cells

### [4-1] Confirmation of Effect in In Vitro

Since NK cells and T cells basically have the activity of killing cancer cells by cell mediation, it was confirmed how the cytolytic activity of NK cells and T cells is affected by the activation of NgR1 as described above.

First, K562 cell line that does not express Nogo-A, which is a ligand of NgR1, was treated with calcein for 1 hour, the K562 cells were stained. The stained cells were co-cultured with NK cells alone or with NK cells plus NEP1-40, which is a (competitive) inhibitor for NgR1, (NK cells:K562 cells = 2:1) for 2 hours. In the co-culture process as described above, when K562 cells were killed by NK cells, calcein was released from the K562 cells into the medium, and the amount of fluorescence of calcein present in the medium was measured using a fluorescence analyzer to determine the degree of K562 cell apoptosis (cytolysis), such that the relative cytolytic activity of NK cells was confirmed. As a result, as illustrated in FIG. 4A, for the K562 cell line that does not express Nogo-A, it was confirmed that there was no significant difference in the cytolytic activity of NK cells with or without the treatment with an inhibitor for NgR1.

In addition, in the same manner as above, K562 cell line that does not express Nogo-A and K562 cell line that overexpresses Nogo-A were stained with calcein, NK cells were treated with K562 cells at a ratio of 2:1 and co-cultured for 2 hours, and then, the degree of K562 cell apoptosis (cytolysis) was confirmed accordingly.

As a result, as illustrated in FIG. 4B, it was confirmed that the fluorescence of calcein was significantly reduced in the K562 cell line overexpressing Nogo-A, whereas when treated with an inhibitor for NgR1, the fluorescence of calcein was restored to a level similar to that in the K562 cell line not overexpressing Nogo-A. From this, it can be seen that the overexpression of Nogo-A negatively affects the cytolytic activity of NK cells against the K562 cell line.

Meanwhile, in the same manner as above, U87MG cells, which are a glioblastoma cell line previously reported to express Nogo-A, were stained with calcein, and then, only NK cells were treated or NK cells were treated together with NEP1-40, which is a (competitive) inhibitor for NgR1, (NK cells:U87MG cells = 2:1), and co-cultured for 2 hours. Then, calcein released into the medium from U87MG cells killed by NK cells was obtained and fluorescence thereof was measured to confirm a degree of apoptosis (cytolysis) of the U87MG cells.

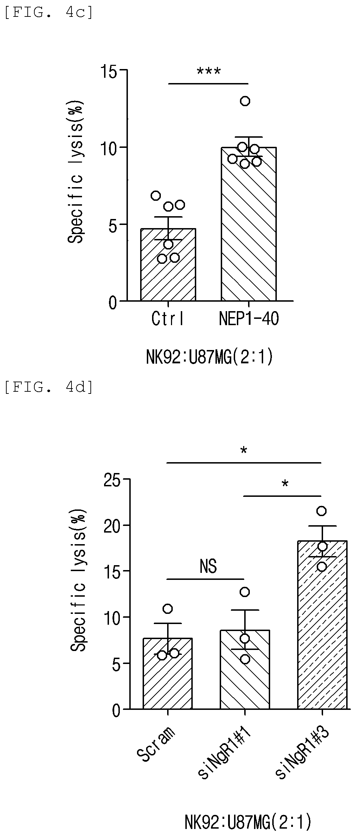

As a result, as illustrated in FIG. 4C, it was confirmed that the fluorescence of calcein was significantly increased when the activation of NgR1 was inhibited by co-treatment with an inhibitor for NgR1 compared to when only NK cells were treated, and from this, it can be seen that the cytolytic activity of NK cells was significantly enhanced when an inhibitor for NgR1 was co-treated.

Furthermore, the U87MG cells stained with calcein as described above were treated with each of NK cells that were simply electroporated, NK cells into which scrambled siRNA (BIONEER Corporation) was introduced by electroporation, or NK cells in which the expression of NgR1 was knocked down by introducing siNgR1 #1 having a base sequence of SEQ ID NO: 97 or siNgR1 #3 having a base sequence of SEQ ID NO: 98, which is siRNA that complementarily binds to mRNA of NgR1, by electroporation (NK cells:U87MG cells = 2:1), and co-cultured for 4 hours. Then, calcein released into the medium from U87MG cells killed by NK cells was obtained and fluorescence thereof was measured to confirm a degree of apoptosis (cytolysis) of the U87MG cells.

As a result, as illustrated in FIG. 4D, it was confirmed that the fluorescence of calcein was significantly increased when NK cells in which the expression of NgR1 was inhibited by the introduction of siRNA were treated, compared to NK cells treated only with electroporation or NK cells into which scrambled siRNA was introduced, and from this, it can be seen that the inhibition of the expression or activity of NgR1 increases the cytolytic activity of NK cells even in cancer cells that express Nogo-A.

Finally, it was confirmed the effect of the activation of NgR1 on the cytolytic activity of T cells using the same method as above. To this end, FBL-3 cells (FBLgag), which are a mouse leukemia cell line expressing gag antigen and Nogo-A, and FBL-3 cells expressing a mutant of gag antigen and Nogo-A (FBLgagΔ) were obtained. In addition, T cells were differentiated by co-culturing splenocytes of transgenic mice with T cells that may specifically recognize gag antigen with IL-2 added to the FBLgag irradiated with radiation for 4 days. In the case of co-culturing splenocytes and FBLgag to differentiate T cells as described above, after 4 days of differentiation, FBLgag is mixed with the T cells, making it difficult to obtain only T cells. Therefore, FBLgag was irradiated with radiation to gradually kill FBLgag, and only T cells were allowed to remain after 4 days of differentiation as described above. Meanwhile, the T cells differentiated as described above may specifically recognize gag antigen, and were co-cultured with calcein-stained FBLgag or FBLgagΔ for 4 hours under conditions with or without treatment with NEP1-40, which is a (competitive) inhibitor for NgR1. Then, calcein released into the medium from FBL-3 cells killed by T cells was obtained and fluorescence thereof was measured to confirm a degree of apoptosis (cytolysis) of the FBL-3 cells.

As a result, as illustrated in FIG. 4E, it was confirmed that the fluorescence of calcein was higher in FBLgag cells that may be recognized by T cells compared to FBLgagΔ cells that express a mutant of gag antigen and thus are not recognized by T cells, and it was confirmed that the fluorescence of calcein was further increased when the T cells were treated together with an inhibitor for NgR1 to inhibit the activation of NgR1 in T cells. From this, it can be seen that the cytolytic activity of T cells is significantly enhanced when treated together with an inhibitor for NgR1.

### [4-2] Confirmation of Effect in In Vivo

The effect of enhancing the cytolytic activity of the NK cells according to the inhibition of NgR1 was once again confirmed in vivo. To this end, as illustrated in FIG. 4F, 4 x 10⁶ U87MG cell lines were injected subcutaneously into the right thigh of 6-week-old NSIG male mice to solidify the cancer. After 10 days, 2.5 x 10⁶ NK cells were injected into the tail vein, or 5 ug of NEP1-40 was injected into the tumor the next day. Then, NK cells were injected 14 days after subcutaneous injection of U87MG cells, NEP1-40 was injected 15 days after subcutaneous injection in the same manner as above, and the size of the cancer over time and the survival rate of the mouse model were measured in the xenograft mouse model.

As a result, as illustrated in FIGS. 4G and 4H, it was confirmed that, when NEP1-40 was injected to inhibit the activity of NgR1, the growth of cancer was suppressed, and the survival rate of the mouse was also significantly improved.

### [4-3] Summary

Finally, it was confirmed that when NgR1 is activated, the cytolytic activity of immune cells such as NK cells and T cells is reduced, and when NgR1 is inhibited, the cytolytic activity of these immune cells is restored. Therefore, from the experimental results as above, it can be seen that NgR1 negatively regulates cytolytic activity in immune cells such as NK cells and T cells.

### [Example 5]

### Confirmation of Biological Role of NgR1 in Anticancer Immunity Based on Cytolytic Activity of Natural Killer Cells

In order to confirm the influence of the relationship between NgR1 of NK cells and Nogo-A of cancer cells on anticancer immunity, first, using Cibersort, which is a database for analyzing the profiles of immune cells infiltrated into cancer cells, all cancer patients were divided into a group with a high infiltration of NK cells and a group with a low infiltration of NK cells. Then, the survival rate of cancer patients according to the expression level of Nogo-A (RTN4) in each group was analyzed using TCGA data.

As a result, as illustrated in FIG. 5A, regardless of the degree of NK cell infiltration, the survival rate of cancer patients that expressed high Nogo-A was confirmed to be lower than that of cancers patients that expressed low Nogo-A.

In addition, using Cibersort, which is a database for analyzing the profiles of immune cells infiltrated into cancer cells, all cancer patients were divided into a group with a high infiltration of CD8 T cells and a group with a low infiltration of CD8 T cells. Then, the survival rate of cancer patients according to the expression level of Nogo-A (RTN4) in each group was analyzed using TCGA data.

As a result, as illustrated in FIG. 5B, regardless of the degree of T cell infiltration, the survival rate of cancer patients that expressed high Nogo-A was confirmed to be lower than that of cancers patients that expressed low Nogo-A.

Finally, from the above results, it can be seen that immune cells such as NK cells and T cells do not exhibit sufficient cytolytic activity against cancer expressing Nogo-A, which prevents sufficient anticancer immunity from occurring, ultimately adversely affecting the survival rate of cancer patients.

### [Example 6]

### Confirmation of Effect of Enhancing Cytolytic Activity of Natural Killer Cells Using Novel Anti-NgR1 Antibodies

Based on the above results, antibodies that may effectively inhibit NgR1 were screened, and novel anti-NgR1 antibodies CSA108 and CSA123 were derived. Thereafter, antibody optimization was performed to derive antibodies with increased antigen binding affinity from the novel antibodies.

First, for the antibody optimization, random mutations were induced across the heavy chain variable region and the light chain variable region of the CSA108 antibody and CSA123 antibody using Diversify PCR Random Mutagensis kit (clontech, cat no. 630703), and the resulting PCR product was cloned into a phagemid vector to construct a library. Thereafter, the antigen and antibody phage library were combined for bio-panning, Fc or histidine of the antigen was bound to each of a Protein A-coated plate or a nickel-coated plate, and then, the phage binding to the antigen was recovered and amplified. hNgR1-Fc and hNgR1-his were used as antigens at a concentration of 0.1 to 1 µg/ml during panning, and blocked with PBS buffer containing 2% BSA. After binding the antibody phage and antigen, washing was performed with PBST (0.1% Tween20).

Thereafter, 100 µl of the antigen was added at a concentration of 1 µg/ml to a 96-well immunoplate, coating was performed overnight at 4°C, washing was performed twice with PBST (0.1% Tween20), and the plate was blocked with PBS buffer containing 2% BSA. After blocking, a poly-phage solution was added for each panning round and bound for at room temperature 1 hour, washing was performed three times with PBST (0.1% Tween20), and anti-M13-HRP (Sino biologics, 11973-MM05T-H) was diluted in PBST at a ratio of 1:5000 and bound at room temperature for 1 hour. The plate in which the reaction was completed was washed three times with PBST, TMB solution (BD, 555214) was added to induce color development for 4 minutes, the color development reaction was stopped using 1 N sulfuric acid, and then, the absorbance was measured at 450 nm.

In addition, after panning was completed, a single colony was selected from the three results and inoculated into a 96-deep well plate, cultured for 4 hours, the culture medium was infected with helper phage (VCSM13) and cultured overnight at 30°C, and then, the supernatant was recovered to perform binding ELISA. First, 0.5 µg/ml of hNgR1 protein or 0.5 µg/ml of isotype IgG1 was coated on a 96-well immunoplate at 4°C overnight, washing was performed twice with PBST (0.1% Tween20), and the plate was blocked with PBS buffer containing 2% BSA. After blocking, the supernatant was added and bound at room temperature for 1 hour, and then, washing was performed three times with PBST. After washing, anti-M13-HRP (Sino biologics, 11973-MM05T-H) was diluted in PBST at a ratio of 1:5000 and bound at room temperature for 1 hour. The plate in which the reaction was completed was washed three times with PBST, TMB solution (BD, 555214) was added to induce color development for 4 minutes, the color development reaction was stopped using 1 N sulfuric acid, and then, the absorbance was measured at 450 nm.

A candidate antibody group was transformed to IgG1 form, transiently expressed in HEK293 cells, purified by Protein A affinity chromatography, and used for SPR analysis. In SPR analysis, a KD value was measured using Biacore T200, candidate antibodies were captured on Protein A chips, and then, the antigen protein (hNgR1-his) was bound at different concentrations for measurement.

As a result of SPR analysis, the binding affinity of the CSA108 antibody to the antigen was found to be 23.3 nM, but in the case of the C1HE2E6 antibody and H6HE2L antibody obtained by optimizing the same, the binding affinities to the antigen were found to be 0.194 nM and 1.69 nM, respectively, confirming that the binding affinity to the antigen was significantly increased through the optimization process.

In addition, the binding affinity of the CSA123 antibody to the antigen was found to be 31.54 nM, but in the case of the G8 antibody, S3 antibody, S6 antibody, and D11 antibody obtained by optimizing the same, the binding affinities to the antigen were found to be 4.149 nM, 5.836 nM, 5.513 nM, and 5.327 nM, respectively, confirming that the binding affinity to the antigen was also significantly increased through the optimization process.

The heavy chain variable region (VH) and light chain variable region (VL) sequences of the novel anti-NgR1 antibodies CSA108 and CSA123 and novel antibodies having increased antigen binding affinity by optimizing these antibodies are as shown in Table 1.

**[Table 1]**

| Antibody | Site | Amino acid sequence | SEQ ID NO: |
|---|---|---|---|
| CSA108 | VH | QMQLVQSGGGLEQPGRSLRLSCVTSGFNFNDYAMHWVRQAPGKGLEWVSG ISWNSGAIGYADSVKGRFTVSRDNSKNSLYLQMNSLRAEDTAVYYCARDY YYETSGYYYGFDNWGQGTLVTVSS | 11 |
| | VL | DIQMTQSPSSVSASVGDRVTITCRASQGISRWLAWYQQKPGKAPKLLIYA ASNLQSGVPLRFSGSGSGTDFTLTINSLQPEDSATYYCQQTNNFPYTFGQ GTKVEIKR | 29 |
| C1HE2E6 | VH | QMQLVQSGGGLEQPGRSLRLSCVTSGFNFNDYAMHWVRQAPGKGLEWVSG ISWNSGAIGYADSVKGRFTVSRDNSKNSLYLQMNSLRAEDTAVYYCARDY NYEISGYYYGFDNWGQGTLVTVSS | 13 |
| | VL | DIQMTQSPSSVSASVGDRVTITCRASQGISRWLSWYQQKPGKAPKLLIYA ASNLQSGVPLRFSGSGFGTDFTLTINSLQPEDSATYYCQQTNNFPYTFGQ GTKVEIKR | 31 |
| H6HE2L | VH | QMQLVQSGGGLEQPGRSLRLSCVTSGFNFNDYAMHWVRQAPGKGLEWVSG ISWNSGAIGYADSVKGRFTVSRDNSKNSLYLQMNSLRAEDTAVYYCARDY NYETSGYYHGFDNWGQGTLVTVSS | 15 |
| | VL | DIQMTQSPSSVSASVGDRVTITCRASQGISRWLSWYQQKPGKAPKLLIYA ASNLQSGVPLRFSGSGSGTDFTLTINSLQPEDSATYYCQQTNNFPYTFGQ GTKVEIKR | 33 |
| CSA123 | VH | QMQLVQSGAEVKKPGASVKVSCKVSGYTLSELSIHWVRQAPGKGLEWMGG FDPEDDETVYAQKFQGRVTLTDDTSTDTAYMELSSLRSEDTAVYYCARDL GYLTSGNYYDAFDIWGRGTMITVSS | 59 |
| | VL | DIQMTQSPSSVSASVGDRVTITCRASQDINRWLAWYQQKPGKAPKLLIYA ASRLQSGVPLRFSGSGSGTDFTLTINSLQPEDSATYYCQQTNNFPRTFGQ GTRLEIKR | 91 |
| G8 | VH | QMQLVQSGAEVKKPGASVKVSCKVSGYPLSELSIHWVRQAPGKGLEWMGG FDSEDDETVYAQKFQGRVTLTDDTSTDTAYMELSSLRSEDTAVYYCARDL GYLTSGNYYDAFGIWGRGTMITVSS | 61 |
| | VL | DIQMTQSPSSVSASVGDRVTITCRASQDINRWLAWYQQKPGKAPKLLIYA ASRLQSGVPLRFSGSGSGTDFTLTINSLQPEDSATYYCQQTNNFPRTFGQ GTRLEIKR | 91 |
| S3 | VH | QMQLVQSGAEVKKPGASVKVSCKVSGYTLSELSIHWVRQAPGKGLEWMGG FDPEDDETVYAQKFQGRVTLTDDTSTDTAYMELSSLRSEDTAVYYCARDL GYLTSGNYYNAFGIWGRGTMITVSS | 63 |
| | VL | DIQMTQSPSSVSASVGDRVTITCRASQDINRWLAWYQQKPGKAPKLLIYA ASRLQSGVPLRFSGSGSGTDFTLTINSLQPEDSATYYCQQTNNFPRTFGQ GTRLEIKR | 91 |
| S6 | VH | QMQLVQSGAEVKKPGASVKVSCKVSGYTLSGLSIHWVRQAPGKGLEWMGG FDSEDDETVYAQKFQGRVTLTDDTSTDTAYMELSSLRSEDTAVYYCARDL GYLTSGNYYDAFDIWGRGTMITVSS | 65 |
| | VL | DIQMTQSPSSVSASVGDRVTITCRASQDINRWLAWYQQKPGKAPKLLIYA ASRLQSGVPLRFSGSGSGTDFTLTINSLQPEDSATYYCQQTNNFPRTFGQ GTRLEIKR | 91 |
| D11 | VH | QMQLVQSGAEVKKPGASVKVSCKVSGYTLSELSIHWVRQAPGKGLEWMGG FDSEDDETVYAQKFQGRVTLTDDTSTDTAYMELSSLRSEDTAVYYCARDL GYLTSGNYYDAFDFWGRGTMITVSS | 67 |
| | VL | DIQMTQSPSSVSASVGDRVTITCRASQDINRWLAWYQQKPGKAPKLLIYA ASRLQSGVPLRFSGSGSGTDFTLTINSLQPEDSATYYCQQTNNFPRTFGQ GTRLEIKR | 91 |

As described above, solutions containing the CSA108 antibody and two types of anti-NgR1 antibodies obtained by optimizing the same, and the CSA123 antibody and four types of anti-NgR1 antibodies obtained by optimizing the same were flowed at different concentrations onto biochips on which the antigen NgR1 was immobilized, and then, surface plasmon resonance analysis of the anti-NgR1 antibodies was performed using a Biacore^{™} 8K system. As a result, as illustrated in FIGS. 6A and 6B, all of the eight types of anti-NgR1 antibodies were confirmed to form specific binding to NgR1, which is an antigen, in a concentration-dependent manner.

Next, instead of NEP1-40, the cytolytic activity of NK cells was analyzed using the CSA108 antibody and two types of anti-NgR1 antibodies obtained by optimizing the same, and the CSA123 antibody and four types of anti-NgR1 antibodies obtained by optimizing the same, and using U87MG cells as target cells in the same manner as in Example [4-1].

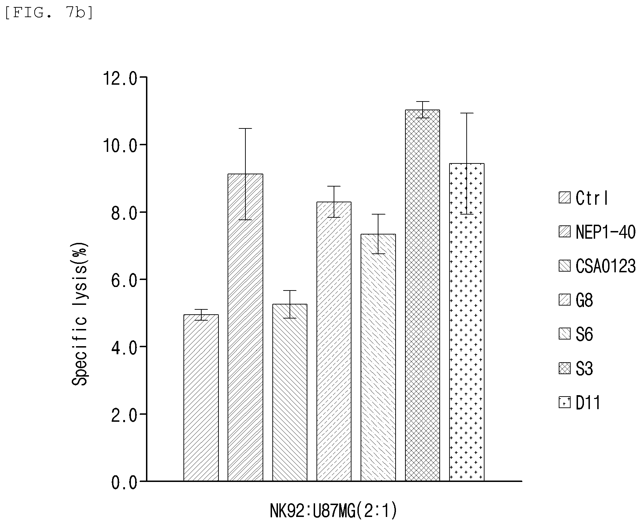

As a result, as illustrated in FIGS. 7A and 7B, it was confirmed that the cytolytic activity of NK cells against target cells (U87MG cells) was enhanced by treatment with the eight types of novel anti-NgR1 antibodies.

Furthermore, instead of NEP1-40, the CSA108 antibody and two types of anti-NgR1 antibodies obtained by optimizing the same were used, and the control DNA was introduced in the same manner as in Example [4-1] to analyze the cytolytic activity of NK cells using K562 cell line that does not express Nogo-A (Mock-K562) and K562 cell line that overexpresses Nogo-A (NogoA-K562) as target cells.

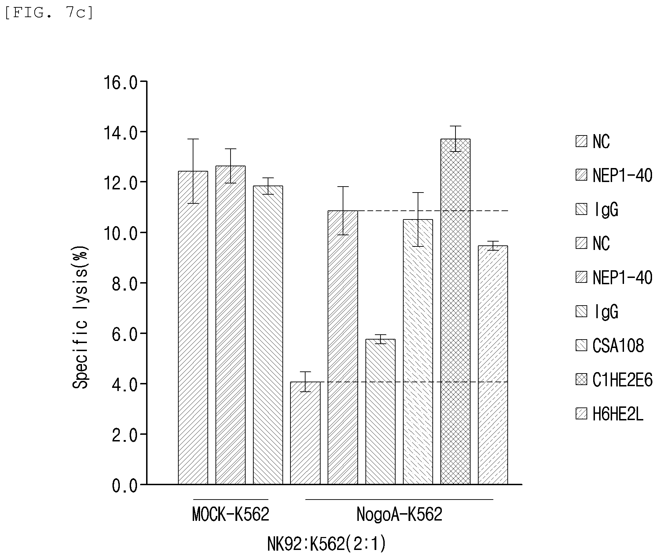

As a result, as illustrated in FIG. 7C, it was confirmed that the cytolytic activity of NK cells against target cells (NogoA-K562) was enhanced by treatment with the three types of novel anti-NgR1 antibodies.

This is the same result as when the activity of NgR1 was inhibited using NEP1-40, which is a (competitive) inhibitor for NgR1, or when the expression of NgR1 was inhibited using siRNA that complementarily binds to the mRNA of NgR1, showing that inhibitor for the NgR1 protein may be composed not only in the form of a peptide or siRNA, but also in the form of an antibody.

### [Example 7]

### Confirmation of Effect of Enhancing Cytolytic Activity of Natural Killer Cells Using Novel siRNA That Specifically Binds to mRNA of NgR1

Meanwhile, through siRNA screening, eight types of novel anti-NgR1 siRNAs having the base sequences shown in Table 2 and specifically binding to mRNA of NgR1 were derived.

**[Table 2]**

| siRNA | Base sequence | SEQ ID NO: |
|---|---|---|
| siNgR1 #11 | CCACCCAGAUCGCGGUAUA | 99 |
| siNgR1 #12 | GCAUCUUCCUGCACGGCAA | 100 |
| siNgR1 #13 | CGAAUGUGCUGGCCCGAAU | 101 |
| siNgR1 #14 | GAGCCCAAGGUGACGACAA | 102 |
| siNgR1 #15 | CGGCAGGCAAUGCGCUGAA | 103 |
| siNgR1 #16 | CAACAAUCUAUCAGCGCUG | 104 |
| siNgR1 #17 | AGGCCAGGCUGUUCACGCA | 105 |
| siNgR1 #18 | AAAUAUCGGACGACGUGGA | 106 |

In the same manner as in Example [4-1], U87MG cells stained with calcein were treated with each of NK cells that were simply electroporated, NK cells co-cultured with NEP1-40, which is a (competitive) inhibitor for NgR1, NK cells into which scrambled siRNA (BIONEER Corporation) was introduced by electroporation, or NK cells in which the expression of NgR1 was knocked down by introducing each of the eight types of siRNAs shown in Table 2 by electroporation (NK cells:U87MG cells = 5:1), and co-cultured for 4 hours. Then, calcein released into the medium from U87MG cells killed by NK cells was obtained and fluorescence thereof was measured to confirm a degree of apoptosis (cytolysis) of the U87MG cells.

As a result, as illustrated in FIG. 8, it was confirmed that the fluorescence of calcein was significantly increased when NK cells in which the expression of NgR1 was inhibited by the introduction of eight types of novel siRNAs were treated, compared to NK cells simply treated with electroporation or NK cells introduced with scrambled siRNA or co-cultured with NEP1-40, and from this, it can be seen that the inhibition of the expression or activity of NgR1 increases the cytolytic activity of NK cells even in cancer cells that express Nogo-A.

### [Example 8]

### Confirmation of Effect of Enhancing Cytolytic Activity of Natural Killer Cells Using Novel Antisense Oligonucleotide (ASO) That Specifically Binds to mRNA of NgR1

In addition, through ASO screening, four types of novel anti-NgR1 ASOs having the base sequences shown in Table 3 and specifically binding to mRNA of NgR1 were derived.

**[Table 3]**

| siRNA | Base sequence | SEQ ID NO: |
|---|---|---|
| aso-NgR1 #2s | gcatcttcctgcacggcaa | 107 |
| aso-NgR1 #3s | cgaatgtgctggcccgaat | 108 |
| aso-NgR1 #5s | cggcaggcaatgcgctgaa | 109 |
| aso-NgR1 #7s | aggccaggctgttcacgca | 110 |

First, after introducing each of the four types of ASOs shown in Table 3 into NK cells, the expression level of NgR1 on the surfaces of the NK cells was measured. As a result, as illustrated in FIG. 9, it was confirmed that the expression of NgR1 was significantly reduced on the surfaces of the NK cells introduced with the four types of novel ASOs compared to the surfaces of the NK cells introduced with the scrambled control.

Next, in the same manner as in Example [4-1], U87MG cells stained with calcein were treated with each of NK cells that were simply electroporated, NK cells co-cultured with NEP1-40, which is a (competitive) inhibitor for NgR1, NK cells into which scrambled control was introduced by electroporation, or NK cells in which the expression of NgR1 was knocked down by introducing each of the four types of ASOs shown in Table 3 by electroporation (NK cells:U87MG cells = 5:1), and co-cultured for 4 hours. Then, calcein released into the medium from U87MG cells killed by NK cells was obtained and fluorescence thereof was measured to confirm a degree of apoptosis (cytolysis) of the U87MG cells.

As a result, as illustrated in FIG. 10, it was confirmed that the fluorescence of calcein was significantly increased when NK cells in which the expression of NgR1 was inhibited by the introduction of four types of novel ASOs were treated, compared to NK cells simply treated with electroporation or NK cells introduced with scrambled ASO or co-cultured with NEP1-40, and from this, it can be seen that the inhibition of the expression or activity of NgR1 increases the cytolytic activity of NK cells even in cancer cells that express Nogo-A.

Although the preferred embodiments of the present invention have been described for illustrative purposes, the scope of the present invention is not limited to the specific embodiments described above, and those skilled in the art will appreciate that modifications are possible within the scope as described in the claims of the present invention.

## Claims

1. An antibody or an antigen-binding fragment thereof that specifically binds to NgR1 protein, the antibody or the antigen-binding fragment thereof comprising:
a heavy chain variable region comprising a CDR1-H comprising an amino acid sequence of SEQ ID NO: 1, SEQ ID NO: 41, SEQ ID NO: 43, or SEQ ID NO: 45, a CDR2-H comprising an amino acid sequence of SEQ ID NO: 3, SEQ ID NO: 47, or SEQ ID NO: 49, and a CDR3-H comprising an amino acid sequence of SEQ ID NO: 5, SEQ ID NO: 7, SEQ ID NO: 9, SEQ ID NO: 51, SEQ ID NO: 53, SEQ ID NO: 55, or SEQ ID NO: 57; and
a light chain variable region comprising a CDR1-L comprising an amino acid sequence of SEQ ID NO: 23, SEQ ID NO: 79, or SEQ ID NO: 81, a CDR2-L comprising an amino acid sequence of SEQ ID NO: 25, SEQ ID NO: 83, or SEQ ID NO: 85, and a CDR3-L comprising an amino acid sequence of SEQ ID NO: 27, SEQ ID NO: 87, or SEQ ID NO: 89.

2. An antibody or an antigen-binding fragment thereof that specifically binds to NgR1 protein, the antibody or the antigen-binding fragment thereof comprising:
a heavy chain variable region comprising an amino acid sequence of SEQ ID NO: 11, SEQ ID NO: 13, SEQ ID NO: 15, SEQ ID NO: 59, SEQ ID NO: 61, SEQ ID NO: 63, SEQ ID NO: 65, or SEQ ID NO: 67; and
a light chain variable region comprising an amino acid sequence of SEQ ID NO: 29, SEQ ID NO: 31, SEQ ID NO: 33, or SEQ ID NO: 91.

3. An antibody or an antigen-binding fragment thereof that specifically binds to NgR1 protein, the antibody or the antigen-binding fragment thereof comprising:
a heavy chain comprising an amino acid sequence of SEQ ID NO: 17, SEQ ID NO: 19, SEQ ID NO: 21, SEQ ID NO: 69, SEQ ID NO: 71, SEQ ID NO: 73, SEQ ID NO: 75, or SEQ ID NO: 77; and
a light chain comprising an amino acid sequence of SEQ ID NO: 35, SEQ ID NO: 37, SEQ ID NO: 39, or SEQ ID NO: 94.

4. The antibody or the antigen-binding fragment thereof of any one of claims 1 to 3, wherein the antigen-binding fragment of the antibody is a single chain variable fragment (scFv).

5. A gene encoding the antibody or the antigen-binding fragment thereof of any one of claims 1 to 3.

6. A recombinant expression vector comprising the gene of claim 5.

7. A transformant in which the recombinant expression vector of claim 6 is introduced into a host cell.

8. A small interfering RNA comprising a polynucleotide sequence of any one of SEQ ID NO: 99 to SEQ ID NO: 106 and specifically binding to NgR1 mRNA.

9. An antisense oligonucleotide comprising a polynucleotide sequence of any one of SEQ ID NO: 107 to SEQ ID NO: 110 and specifically binding to NgR1 mRNA.

10. A composition for enhancing cytolytic activity of immune cells, the composition comprising, as an active ingredient, one or more selected from the group consisting of the antibody or the antigen-binding fragment thereof of any one of claims 1 to 3, the small interfering RNA of claim 8, and the antisense oligonucleotide of claim **9.**

11. The composition of claim 10, wherein the composition enhances activity of the immune cells by promoting formation of immunological synapses of the immune cells or regulating the immune cells.

12. The composition of claim 10, wherein the composition is a pharmaceutical composition for preventing or treating an NgR1-associated disease, and
the NgR1-associated disease is one or more selected from the group consisting of cancer, multiple sclerosis, ALS, Huntington's disease, Alzheimer's disease, Parkinson's disease, diabetic neuropathy, stroke, traumatic brain injury, and spinal cord injury.

13. The composition of claim 12, wherein the cancer is one or more selected from the group consisting of skin cancer, breast cancer, uterine cancer, esophageal cancer, stomach cancer, brain tumor, glioblastoma, colon cancer, rectal cancer, lung cancer, ovarian cancer, cervical cancer, endometrial cancer, vulvar cancer, kidney cancer, blood cancer, pancreatic cancer, prostate cancer, testicular cancer, laryngeal cancer, head and neck cancer, thyroid cancer, liver cancer, bladder cancer, osteosarcoma, lymphoma, blood cancer, thymus cancer, urethral cancer, and bronchial cancer.

14. The composition of claim 13, further comprising a cancer immunotherapeutic agent.

15. The composition of claim 14, wherein the cancer immunotherapeutic agent is one or more selected from the group consisting of anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-CD73, anti-ICOS, anti-CD40L, anti-BTLA, anti-TCR, anti-TIGIT, anti-VISTA, anti-TIGIT, and anti-NKG2A.

16. The composition of claim 10, wherein the immune cells are natural killer cells, T cells, or natural killer T cells.

17. The composition of claim 10, further comprising NEP1-40.

18. A chimeric antigen receptor comprising the antibody or the antigen-binding fragment thereof of any one of claims 1 to 3.

19. An immune cell expressing the chimeric antigen receptor of claim 18.
